Europäisches Patentamt

European Patent Office

Office européen des brevets

(19)

(11) EP 1 598 009 A1

(12) EUROPEAN PATENT APPLICATION
published in accordance with Art. 158(3) EPC

(43) Date of publication:
23.11.2005 Bulletin 2005/47

(51) Int Cl.7: A61B 5/117

(21) Application number: 04715505.6

(86) International application number:
PCT/JP2004/002378

(22) Date of filing: 27.02.2004

(87) International publication number:
WO 2004/075751 (10.09.2004 Gazette 2004/37)

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 27.02.2003 JP 2003051869

(71) Applicant: SONY CORPORATION
Tokyo 141-0001 (JP)

(72) Inventor: Takiguchi, Kiyoaki,
c/o Sony Corporation
Tokyo 141-0001 (JP)

(74) Representative: Mills, Julia et al
D Young & Co
120 Holborn
London EC1N 2DY (GB)

(54) AUTHENTICATION SYSTEM

(57) The present invention enables the degree of freedom in communication to be enhanced. According to the present invention, in an authentication system 1, during a walking motion of a user which is essentially required for the user to pass through an entrance/exit passage portion 4 (Figure 6), tissue information D5 and walking information D7 as living organism identification information, a pattern specific to a user, is detected from an detection electrode 52 via an FET 11, based on a walking quasi-electrostatic field HSE formed in the neighborhood of the user. Thereby, information with high identifiability specific to the user can be obtained without controlling movements of the user and without performing special processing such as encryption. Thus, the degree of freedom in communication can be enhanced.

10 ELECTRIC FIELD DETECTION PORTION                    3

20 SENDING PORTION                    ~D6

A1~An

TISSUE INFORMATION
GENERATION PORTION    21

14 AMPLIFIER  S1                    ~D5

22 S2        23                    24

LPF    WAVEFORM        MODULATION
        PROCESSING PORTION  CIRCUIT

                                D4, D7

                                ~HS

15 CASE    30 ELECTRIFICATION INDUCTION PORTION

11 FET    31 ELECTRIFICATION
          INDUCIOTN ELECTORDE

          ~32 DIELECTRIC

12 DETECTION ELECTRODE

13 DIELECTRIC

OS USER'S EPIDERMIS

FIG. 7

EP 1 598 009 A1

**Description**

Technical Field

The present invention relates to a communication system and is preferably applicable to a communication system for sending and receiving information via an electric field, for example.

Background Art

Conventionally, communication systems have been adapted to send and receive information using a radiation field (radio waves), for example, between mobile telephones, and send and receive information via electromagnetic induction, for example, between the coil in a data reader/writer provided on a ticket checking and collecting machine at a station and the coil in an IC card.

Recently, there have been proposed communication systems which are provided with a human-body-side communication device fitted in contact with the skin of a human body and an equipment-side communication device in the neighborhood of the user as shown in Table 1 below. In these communication systems, an alternating voltage is applied to the human body via the electrode of the human-body-side communication device, and as a result, there is caused an electrostatic induction phenomenon at the electrode of the equipment-side communication device by the action of a capacitor using a human body intervening between the electrodes of the communication device on the human body side and the communication device on the equipment side as a medium. Using the electrostatic induction phenomenon, information is sent and received (see Non-patent document 1, for example).

At least two leads (a signal line and a reference line) are required for performing wired communication between equipment A and B.

Equipment A — Signal line / Reference line — Equipment B

How should the two lines arranged when a human body is utilized as a lead?

⇕

A human body is one lead

| | Our company's method | Company A's method | Company B's method |
|---|---|---|---|
| Basic principle | Communication device on the humanbody side / Communication deviceon the equipment side / Static coupling<br><br>Signal line: Utilizes the human body<br>Reference line: Utilizes the human body and static coupling between the human body and the ground | Communication device on the humanbody side / Communication deviceon the equipment side / Static coupling<br><br>Signal line: Utilizes the human body<br>Reference line: Utilizes static coupling between the electrode of the communication device on the human body side and the ground | Communication device on the humanbody side / Static coupling / Communication deviceon the equipment side<br><br>□ : Signal electrode  ▭ : Reference electrode  — : Signal line  --- : Reference line<br><br>Signal line: Utilizes the human body<br>Reference line: Utilizes static coupling between the electrodes of the communication device on the human body side and the communication device on the equipment side |
| Characteristics | ○:Stable because the distance during which unstable static coupling is utilized is short. External noises influence little.<br>○:The electrode area can be reduced and miniaturization is possible.<br>△:The way of mounting is restricted (directional restriction). | ×:Unstable because the distance during which static coupling is utilized is long. Easily influenced by external noises.<br>△:A large electrode area is required. Miniaturization is difficult.<br>○:The degree of freedom in mounting is high. | ×:Unstable because the strength of static coupling varies according to use conditions.<br>△:The distance between the communication device on the human body side and the communication device on the equipment side can not be lengthened. |

In addition to the communication systems shown in Table 1, there have been proposed a lot of communication systems adapted to send and receive information utilizing the electrostatic induction phenomenon caused at a receiving electrode by the action of a capacitor using a human body intervening between sending and receiving electrodes as a medium (see Patent documents 1 to 9 and Non-patent documents 2 to 5).

[Patent document 1] National Publication of International Patent Application No. 11-509380

[Patent document 2] Patent No. 3074644

[Patent document 3] Japanese Patent Laid-Open No. 10-228524

[Patent document 4] Japanese Patent Laid-Open No. 10-229357

[Patent document 5] Japanese Patent Laid-Open No. 2001-308803

[Patent document 6] Japanese Patent Laid-Open No. 2000-224083

[Patent document 7] Japanese Patent Laid-Open No. 2001-223649

[Patent document 8] Japanese Patent Laid-Open No. 2001-308803

[Patent document 9] Japanese Patent Laid-Open No. 2002-9710

[Non-patent document 1] Internet <URL:http://www.mew.co.jp/press/0103/0103-7.htm> (retrieved on January 20, 2003)

[Non-patent document 2] "Development of Information Communication Device with Human Body Used as Transmission Line" by Keisuke Hachisuka, Anri Nakata, Kenji Shiba, Ken Sasaki, Hiroshi Hosaka and Kiyoshi Itao (Tokyo University); March 1, 2002 (Collected Papers for Academic Lectures on Micromechatronics, Vol., 2002, Spring, pp. 27-28)

[Non-patent document 3] "Development of Communication System within organism" by Anri Nakata; Keisuke Hachisuka, Kenji Shiba, Ken Sasaki, Hiroshi Hosaka and Kiyoshi Itao (Tokyo University); 2002 (Collected Papers for Academic Lectures for Japan Society of Precision Engineering Conference, Spring, p.640)

[Non-patent document 4] "Review on Modeling of Communication System Utilizing Human Body as Transmission Line" by Katsuyuki Fujii (Chiba University), Koichi Date (Chiba University), Shigeru Tajima (Sony Computer Science Laboratories, Inc.); March 1, 2002 (Technical Reports by The Institute of Image Information and Television Engineers Vol. 26, No. 20, pp. 13-18)

[Non-patent document 5] "Development of Information Communication Device with Human Body Used as Transmission Line" by Keisuke Hachisuka, Anri Nakata, Kento Takeda, Ken Sasaki, Hiroshi Hosaka, Kiyoshi Itao (Graduate School of Science of NewRegion Creation, Tokyo University) and Kenji Shiba (Science and Engineering Course, Tokyo University of Science); March 18, 2002 (Micromechatronics Vol. 46; No. 2; pp. 53-64)

In these communication systems with such a configuration, since the action of a capacitor using a human body intervening between sending and receiving electrodes as a medium is the premise of physical action, the communication strength in communication between the electrodes depends on the area of the electrodes.

Furthermore, since the action of a capacitor using a human body intervening between sending and receiving electrodes as a medium is the premise of physical action, it is physically impossible, when the sending electrode is fitted to the human's right wrist, for example, to communicate in directions other than the direction from the human' s right wrist to the fingertip. When the sending electrode is fitted near the human's chest, communication in directions other than the forward direction from the human's chest is physically impossible.

As described above, in communication systems, since the action of a capacitor using a human body intervening between sending and receiving electrodes as a medium is the premise of physical action, there have been a problemthat the communication direction is restricted by the position of the electrode fitted to a human body as well as a problem that the degree of freedom in communication is low because the communication strength depends on the electrode area.

Disclosure of the Invention

The present invention has been made in consideration of the above problems and proposes a communication system, a communication method and a communication device capable of enhancing the degree of freedom in communication.

According to the present invention, for the purpose of solving the above problems, in an authentication system comprising a sending device and a receiving device for sending and receiving information via a quasi-electrostatic field, the sending device is adapted to detect organism information specific to a human body, generate a quasi-electrostatic field modulated according to the organism information and thereby electrify the human body, while the receiving device is adapted to demodulate the organism information based on change in the electrification condition of the human body and identify the human body based on the organism information.

Consequently, the authentication system is able to realize sending and receiving of information without directional restrictions in the neighborhood of the user, with confidentiality secured, and without forcing the user to perform a predetermined movement, and it is also able to identify even the relation between the device and the human body based on information specific to the human body.

Brief Description of the Drawings

Figure 1 is a schematic diagram provided to explain a polar coordinate system;
Figure 2 is a graph showing relative strength change (1) of each electric field relative to the distance;
Figure 3 is a graph showing relative strength change (2) of each electric field relative to the distance;
Figure 4 is a graph showing the relation between the wavelength and the distance;
Figure 5 is a schematic diagram provided to explain the tissue of a human body;
Figure 6 is a schematic diagram showing the entire configuration of an authentication system to which the present invention is applied;
Figure 7 is a schematic block diagram showing the configuration of a card device;
Figure 8 is a schematic diagram showing the configuration of a microelectrode;
Figure 9 is a block diagram showing the configuration of a waveform processing portion;
Figure 10 is a schematic diagram provided to explain a walking waveform;
Figure 11 is a schematic diagram provided to explain cutout and division of a waveform;
Figure 12 is a flowchart showing a procedure for a sending process;
Figure 13 is a flowchart showing a procedure for a masking time determination process;
Figure 14 is a flowchart showing a procedure for a walking information generation process;
Figure 15 is a schematic block diagram showing the configuration of an authentication device;
Figure 16 is a graph provided to explain integral values for subdivided sections;
Figure 17 is a flowchart showing a procedure for an authentication process;
Figure 18 is a flowchart showing a procedure for a walking waveform checking process;

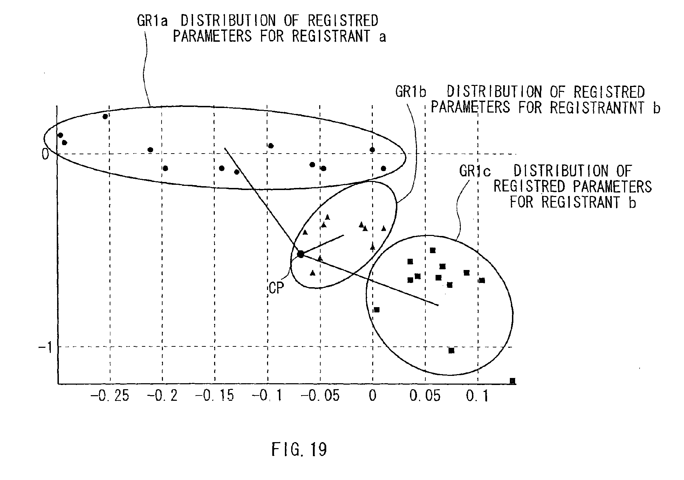

Figure 19 is a schematic diagram provided to explain an example of calculating the Mahalanobis' distance;
Figure 20 is a schematic diagram provided to explain the floor surface of the authentication device;
Figure 21 is a schematic diagram showing the equipotential surface of a quasi-electrostatic field to be formed when a human body is caused to act as an ideal dipole antenna;
Figure 22 is a schematic diagram showing the equipotential surface of a quasi-electrostatic field performed according to this embodiment;
Figure 23 is a schematic diagram provided to explain prevention of electrical leakage; and
Figure 24 is a schematic diagram showing the configuration of a noise absorption/grounding line.

Best Mode for Carrying Out the Invention

The present invention is now described in detail with reference to the drawings.

(1) Summary of the invention

According to the invention, information is sent and received using an electric field. The summary of the present invention is now described in terms of the relation with the electric field.

(1-1) Electric field

Generally, when current flows through an electric dipole (dipole antenna), the electric field E generated according to the distance r from the antenna can be represented in a simplified formula as shown below:

$$E_0 = A\left( \frac{1}{r^3} + \frac{jk}{r^2} + \frac{k^2}{r^1} \right)$$

$$\ldots \ldots (1)$$

where j is an imaginary unit, A is a constant, and k is the number of waves.
As shown in the above formula (1), the electric field E can be roughly separated into a component which is in inverse proportion to the distance r raised to the third power (hereinafter, this component is referred to as a quasi-electrostatic field), a component which is in inverse proportion to the distance r raised to the second power (hereinafter, this component is referred to as an induction field) and a component which is linearly in inverse proportion to the distance r (hereinafter, this component is referred to as a radiation field).

The radiation field is a component excellent in propagation capability, which does not rapidly attenuate even when the distance r is long, since it is only linearly in inverse proportion to the distance r, and therefore, it has been used as a common information transmission medium in the art of information communication.

Though the induction field is a component with little transmission capability, which attenuates in inverse proportion to the distance r raised to the second power as the distance r lengthens, it has recently been used as an information transmission medium in a part of the art of information of communication.

The quasi-electrostatic field is a component which rapidly attenuates in inverse proportion to the distance r raised to the third power and therefore does not a transmission capability and which appears in close proximity to an oscillation source only as oscillation. Therefore, it has not been utilized in the art of information communication where the radiation field and the induction field are premises.

The present invention is adapted to send and receive information within a neighbor communication range, with a neighbor communication (hereinafter referred to as near field communication) approach using a quasi-electrostatic field among electric fields.

(1-2) Quasi-electrostatic field

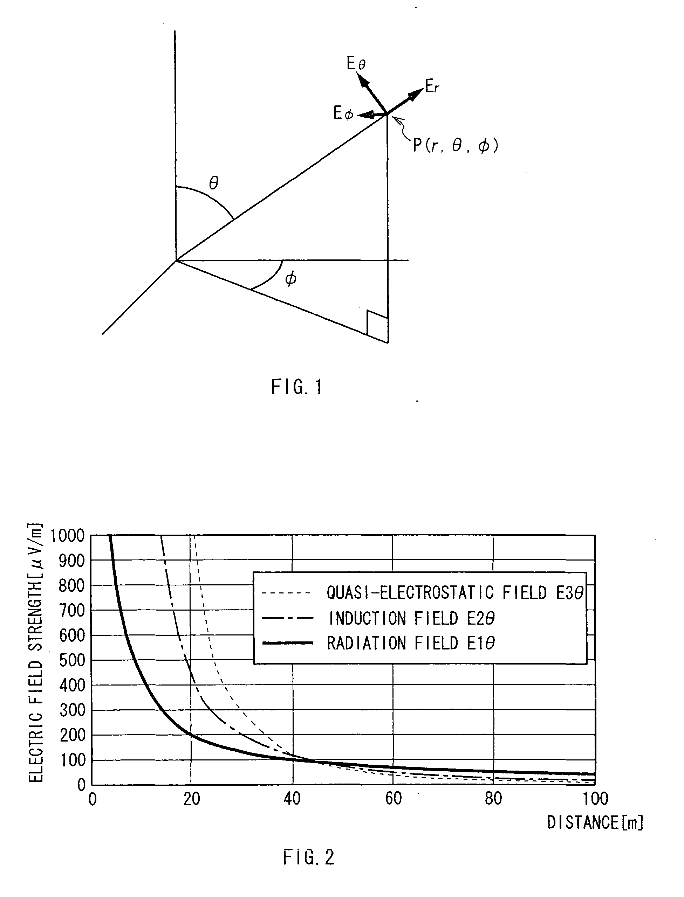

The quasi-electrostatic field is now described in more detail. First, the electric field E shown in the above formula (1) is represented as an electric field at a position P $(r, \theta, \phi)$ at a predetermined distance from the origin as described in Figure 1.

In this case, if it is assumed that a charge q and a charge -q exist separated by a distance $\delta$ and the charge q changes to "$Q\cos\omega t$" at a time t, then the electric fields Er, E$\theta$ and E$\phi$ at the position P $(r, \theta, \phi)$ can be represented as the following formulas, respectively, with the position of the charge q as the origin:

$$E_r = \frac{Q\cos\omega t\, \sigma \cos\theta}{2\pi\varepsilon r^3}(1 + jkr)\exp(-jkr)$$

$$E_\theta = \frac{Q\cos\omega t\, \sigma \sin\theta}{4\pi\varepsilon r^3}(1 + jkr + (jkr)^2)\exp(-jkr)$$

$$E_\phi = 0 \tag{2}$$

In the formulas (2), the electric field E$\phi$ is "zero", and this means that there is not generated any electric field in the $\phi$ direction from the position P (Figure 1).

If the component which is linearly in inverse proportion to the distance r (that is, the radiation field) is separated from the electric fields Er and E$\theta$ represented in the formulas (2), then the radiation field E1r and E1$\theta$ at the position P $(r, \theta, \phi)$ are represented as the following formulas:

$$E_{1r} = 0$$

$$E_{1\theta} = \frac{Q\cos\omega t\, \sigma \sin\theta}{4\pi\varepsilon r}(j^k)^2 \exp(-jkr) \tag{3}$$

If the component which is in inverse proportion to the distance r raised to the second power (that is, the induction field) is separated from the electric fields Er and E$\theta$ represented in the formulas (2), then the induction fields E2r and E2$\theta$ at the position P $(r, \theta, \phi)$ are represented as the following formulas:

$$E_{2r} = \frac{Q\cos\omega t\, \sigma \cos\theta}{2\pi\varepsilon r^2} jk\cdot \exp(-jkr)$$

$$E_{2\theta} = \frac{Q \cos \omega t \, \sigma \sin \theta}{4\pi\varepsilon r^2} \, jk \cdot \exp(-jkr) \tag{4}$$

Furthermore, if the component which is in inverse proportion to the distance r raised to the third power (that is, the quasi-electrostatic field) is separated from the electric fields Er and Eθ represented in the formulas (2), then the quasi-electrostatic fields E3r and E3θ at the position P (r, θ, φ) are represented as the following formulas:

$$E_{3r} = \frac{Q \cos \omega t \, \sigma \cos \theta}{2\pi\varepsilon r^3}$$

$$E_{3\theta} = \frac{Q\cos \omega t \, \sigma \sin \theta}{4\pi\varepsilon r^3} \tag{5}$$

In the formulas (3), only the radiation field E1r is "zero", and this means that the tangent direction components at the position P (Fig. 1) is zero.

Now, in order to show the component's electric field strength of each of the radiation field, the induction field and the quasi-electrostatic field at a distance r, the radiation field E1θ, the induction field E2θ and the quasi-electrostatic field E3θ in the formulas (3) to (5) are now described in more detail.

The number of waves k [m$^{-1}$] is in the relation shown as the following formula, where the angular frequency is denoted by ω and the light velocity is denoted by c:

$$k = \frac{\omega}{c} \tag{6}$$

If the number of waves k is substituted into the formula (6), the "j·exp(-jkr)" is removed since it is beyond the discussion here, and the "cosωt" is assumed to be one (1) since the maximum change with time between the charge q and the charge -q is to be considered, then the following formulas are obtained:

Radiation field

$$E_{1\theta} = \frac{Q \, \sigma \sin \theta}{4\pi\varepsilon r^3} \left(\frac{\omega}{c} r\right)^2$$

Induction field

$$E_{2\theta} = \frac{Q \, \sigma \sin \theta}{4\pi\varepsilon r^3} \, \frac{\omega}{r}$$

Quasi-electrostatic field

$$E_{3\theta} = \frac{Q \, \sigma \sin \theta}{4\pi\varepsilon r^3} \tag{7}$$

If the formulas (7) are rearranged by substituting the distance δ, the charge q (= Q) and the θ with one (1), 0.001 [C] and π/2, respectively, then the following formulas are obtained:

Radiation field

$$E_{1\theta} = \frac{0.001}{4\pi\varepsilon_0 r} \left(\frac{\omega}{c}\right)^2$$

Induction field

$$E_{2\theta} = \frac{0.001}{4\pi\varepsilon_0 r^2} \frac{\omega}{c}$$

Quasi-electrostatic field

$$E_{3\theta} = \frac{0.001}{4\pi\varepsilon_0 r^3} \qquad (8)$$

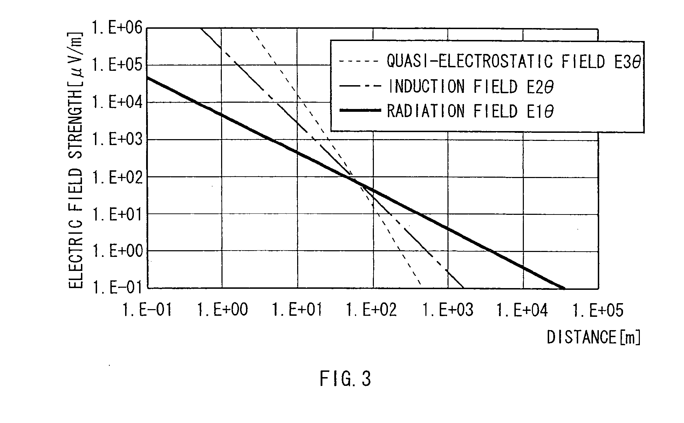

Figures 2 and 3 shows the results obtained by qualitatively plotting the component's electric field strengths of the radiation field $E_{1\theta}$, the induction field $E_{2\theta}$ and the quasi-electrostatic field $E_{3\theta}$ based on the formulas (8).

However, in Figures 2 and 3, the component's electric field strengths at a frequency of 1 [MHz] are shown, and in Figure 3, a relation between component's distance and electric field strength shown in Figure 2 is shown by a graph with a measure of logarithm.

Especially apparent from Figure 3, the component electric field strengths of the radiation field $E_{1\theta}$, the induction field $E_{2\theta}$ and the quasi-electrostatic field $E_{3\theta}$ are equal at a certain distance r (hereinafter referred to as a boundary point), and the radiation field $E_{1\theta}$ is dominant in the distance from the boundary point. On the contrary, in the neighbor before the boundary point, the quasi-electrostatic field $E_{3\theta}$ is dominant.

At the boundary point, the following formula is established according to the above formulas (8):

$$\frac{\omega}{c} \cdot r = 1 \qquad (9)$$

The light velocity c is in the relation shown by the following formula, where the wavelength is denoted by $\lambda$ and the frequency is denoted by f:

$$c = \lambda \cdot f \qquad (10)$$

The angular frequency $\omega$ is in the relation shown by the following formula:

$$\omega = 2\pi f \qquad (11)$$

Then, by substituting the formula (10) and the formula (11) into the formula (9) and rearranging the formula (9), the following formula is obtained:

$$r = \frac{\lambda}{2\pi} \qquad (12)$$

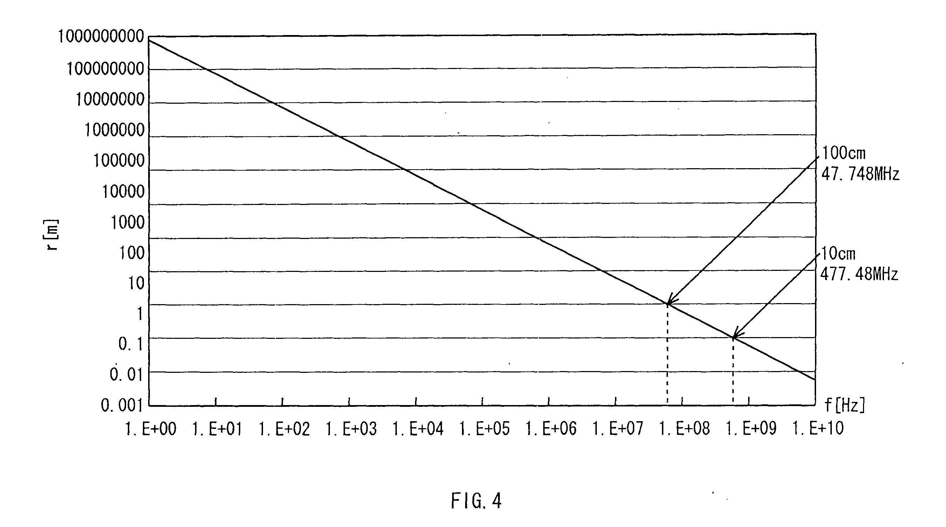

According to the formula (12), the distance r from the origin to the boundary point varies according to the wavelength $\lambda$. As shown in Figure 4, the longer the wavelength $\lambda$ is, the wider the range (the distance r from the origin to the boundary point) where the quasi-electrostatic field $E_{3\theta}$ is dominant.

To sum up the above description, the quasi-electrostatic field $E_{3\theta}$ is dominant within the range where the distance r from the origin is "$r < \lambda/2\pi$", if the relative permittivity of the air $\varepsilon$ is assumed to be 1 and the wavelength in the air is assumed to be $\lambda$.

In the present invention, by selecting the range satisfying the formula (12) when sending and receiving information with the near field communication approach, the information is sent and received in the space where the quasi-electrostatic field $E_{3\theta}$ is dominant.

(1-3) A quasi-electrostatic field and a human body

Though it is necessary to apply current to a human body to cause the human body to generate a radiation field or an induction field, it is physically difficult to efficiently apply current to the human body because the impedance of a human body is very high. It is also physiologically undesirable to apply current to a human body. As for static electricity, however, the situation is completely different.

That is, a human body is very often electrified as suggested by the empirical fact that static electricity is felt in our everyday life. As it is known that a quasi-electrostatic field is generated by electrification of the surface of a human body in response to the movement of the human body, it is not necessary to apply electricity to a human body to cause the human body to generate a quasi-electrostatic field but it is only necessary to electrify the human body.

That is, a human body is electrified by extremely little movement of charge (current); the electrification change is instantaneously conducted around the surface of the human body; and then an equipotential surface of a quasi-electrostatic field is formed substantially isotropically from the periphery. Furthermore, within the range satisfying the above formula (12) where the quasi-electrostatic field is dominant, the radiation field and the induction field does not have much influence. Consequently, the human body functions efficiently as an antenna. This has already been confirmed from the results of the experiments by the applicant.

As a near field communication technology, the present information is adapted to modulate a quasi-electrostatic field which is isotropically formed in the neighborhood of a human body by electrifying the human body according to particular information, and as a result, formaquasi-electrostatic field having information in the neighborhood of the human body, through which the information is sent and received.

(1-4) A quasi-electrostatic field and a walking motion of a human body

As already stated, the surface of a human body is electrified in response to a movement of the human body. Description will be now made on the relation between walking, one of major movements of a human body, and electrification in more detail. Such relation is already disclosed in Japanese Patent Application No. 2002-314920 by the applicant.

That is, as for displacement of the strength of a quasi-electrostatic field formed as the surface of a human body is electrified by the human body's walking motion (hereinafter, it is referred to as a walking quasi-electrostatic field), not only movement of a charge between the passage surface and the plantar surface but also change in the exfoliation area (or the contact area) of the plantar surface relative to the passage surface and change in the distance between the passage surface and the plantar surface are closely involved.

In other words, the electrification change on the surface of a human body caused by a walking motion of the human body reflects a pattern specific to the individual, which is generated by change in the electrostatic capacity and charge between the feet and the passage surface according to the trajectory of the feet made by the walking motion and in which mutual movements of the right and left feet are combined. Thus, the pattern of electrification change on the surface of a human body which is generated by a walking motion of the human body reflects the characteristics of the organism (human body), and therefore high accuracy of authentication can be expected therefrom.

Accordingly, the present invention is adapted to generate organism identification information indicating a walking pattern specific to a human body, based on the pattern of electrification change on the surface of the human body generated by a walking motion of the human body, and use the organism identification information to perform a predetermined authentication process.

At the instant when the tiptoe of the right foot (left foot) has completely left the ground, the left foot (right foot) is completely in contact with the passage surface, irrespective of difference in the walking condition, according to walking characteristics.

Accordingly, in such a condition, mutual electrification action (interference action) between the right and left feet does not occur, and in displacement of strength of the walking quasi-electrostatic field in this condition, the highest peak amplitude appears specifically within the band of 8 Hz±2 Hz.

As for details of the amplitude peak which appears within the band of 8 Hz±2 Hz (hereinafter referred to as the 8 Hz peak) , see Japanese Patent Application No. 2002-314920 (paragraph No. [0024] on p. 5 to paragraph No. [0056] on p.12) already disclosed by the applicant. The walking motion described in the invention means a movement of walking on a flat passage surface without being especially conscious of the speed.

As described above, the 8 Hz peak with the highest strength appears in a walking quasi-electrostatic field formed in the neighborhood of a human bodywhen awalkingmotion is performed, therefore, if attempting to electrify a human body to form a quasi-electrostatic field having information in the neighborhood of a the human body, for the purpose of performing near field communication, the information may be destroyed by the 8 Hz peak.

Therefore, the present invention is adapted to avoid destruction of information by the 8 Hz peak by electrifying a human body according to information while avoiding the timing when such 8 Hz peak appears.

(1-5) A quasi-electrostatic field and the tissue structure of a human body

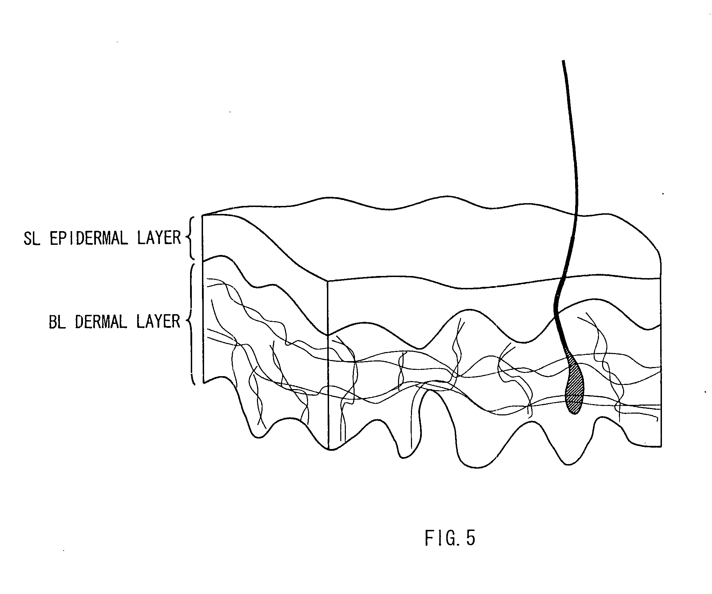

The subepidermal tissue structure of a human body comprises epidermal layers SL and dermal layers BL constituted of epidermoid cells, as shown in Figure 5, and various proteins are contained in the dermal layers BL, such as collagen classified as structural protein and hemoglobin classified as transport protein. It has been already confirmed by the applicant that the pattern of the subepidermal tissue structure is information specific to an individual.

As an approach for detecting the pattern of the subepidermal tissue structure, approaches described are conceivable, for example. In a first approach, the potential of a human body with multiple planar microelectrodes attached to the epidermal surface thereof, that is electrified when it enters a quasi-electrostatic field, is detected by each of the microelectrodes as a relative potential difference.

In a second approach, which utilizes the fact that a human body is electrified by a walking motion thereof as described above, the potential of a human body with multiple planar microelectrodes attached to the epidermal surface thereof, that is electrified when it walks, is detected by each the microelectrodes as a relative potential difference.

In both of the two approaches described above, electrostatic capacity difference between the structure under each microelectrode and the microelectrode is detected. This means that the tissue structure under each microelectrode, that is, the pattern of tissue to be information for identifying an individual is detected. In the two approaches, high-frequency signals for detection are not directly applied to the skin externally, and therefore, it is considered that physiological burden is not imposed on the human body when detection is performed.

For example, if one microelectrode arranged on the surface of a human body is represented by EN (i) , the distance to the deepest layer of the dermal layers BL under the microelectrode EN(i) is represented by md (i) , and the relative permittivity is represented by $\varepsilon$, and the permittivity of vacuum electric constant is represented by $\varepsilon_0$, then the electrostatic capacity C(i) between the microelectrode EN(i) and the deepest layer of the dermal layers BL under the microelectrode EN(i) with the distance md(i) therebetween is represented by the following formula:

$$C\,(i) = \varepsilon\,\varepsilon_0\,\frac{S}{md\,(i)} \tag{13}$$

and the potential V(i) detected by the microelectrode EN(i) when the human body is electrified with a charge Q is represented by the following formula:

$$V\,(\,i\,) = Q \cdot \frac{md\,(i)}{\varepsilon\,\varepsilon_0\,S} \tag{14}$$

Actually, the detected potential is influenced not only by the distance md (i) from the microelectrode EN (i) to the deepest layer of the dermal layers BL but also by the presence of cutaneous veins and the like.

Therefore, it is difficult to accurately determine the distance md(i) with limitation only to blood vessels, for example, included in the dermal layers BL, and the presence of subepidermal blood vessels is also reflected in the measured value. It is known that subepidermal tissue structure indicates characteristics specific to an individual and is permanent in relation to fingerprints, for example, and the same is true on the vein pattern. Accordingly, the potential difference pattern for each microelectrode reflects the characteristics of the living tissue including the subepidermal tissue and veins below the electrode, and therefore high accuracy of authentication can be expected.

Accordingly, the present invention is adapted to generate living organism identifying information indicating a tissue pattern specific to a human body based on a potential difference pattern characterizing the living tissue, and performs a predetermined authentication process using the living organism identifying information.

As described above, the present invention utilizes the nature of a quasi-electrostatic field and the nature of a human body. To sum up the present invention, within a range where a quasi-electrostatic field is dominant, the sending side generates living organism identifying information indicating a walking pattern obtained based on the electrification change on the surface of a human body, which is caused by a walking motion of the human body, and a living tissue pattern; then electrifies the human body to cause the human body to act as an antenna for the purpose of near field communication while avoiding the timing when the 8 Hz peak appears; and, as a result, forms a quasi-electrostatic field having living organism identifying information in the neighborhood of the human body.

Meanwhile, the receiving side, after detecting the living organism identifying information via the quasi-electrostatic field formed in the neighborhood of the human body, performs a predetermined authentication process using registrant information registered in advance. One embodiment to which the present invention is applied will be now described below.

(2) One embodiment of the present invention

(2-1) Entire configuration of an authentication system

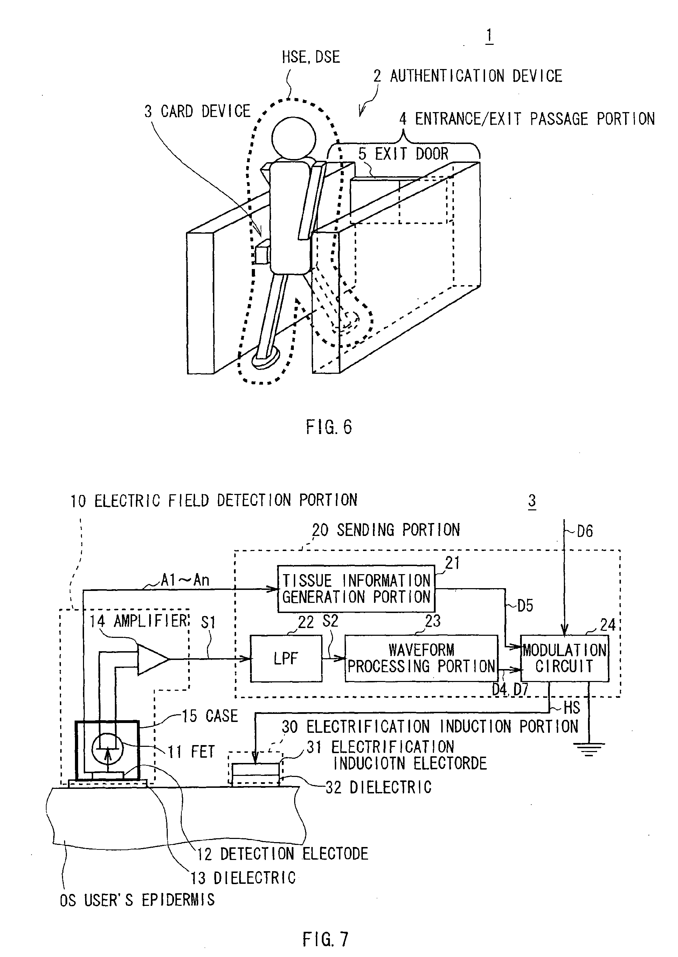

In Figure 6, reference numeral 1 denotes the entire configuration of an authentication system to which the present invention is applied. The authentication systemcomprises an authentication device 2 provided, for example, at the entrance of a company, and a mobile device (hereinafter referred to as a card device ) 3 attachably and detachably provided on a predetermined position of the arm of a human body (hereinafter referred to as a user) that utilizes the company.

The authentication device 2 comprises an entrance/exit passage portion 4 provided for entrance and exit, and an exit door 5 openably and closably provided on the exit side of the entrance/exit passage portion 4, and is adapted to perform near field communication with the card device 3 provided on the user who is passing through the entrance/exit passage portion 4 and open the exit door 5 which is closed, as necessary.

(2-2) Configuration of a card device

As shown in Figure 7, the card device 3 comprises an electric field detection portion 10, a sending portion 20 and an electrification induction portion 30.

The electric field detection portion 10 has a field effect transistor (hereinafter referred to as an FET) 11, and the gate of the FET 11 is connected to the user' s epidermis OS, which is a detection target, via an detection electrode 12 and a dielectric 13 sequentially. The source and the drain of the FET 11 are connected to an amplifier 14.

The electric field detection portion 10 is adapted to detect strength change of a walking quasi-electrostatic field HSE (Figure 7) formed in the neighborhood of a user, which is caused by electrification of the surface of the user coming near to the entrance/exit passage portion 4, via the dielectric 13 and the detection electrode 12 sequentially, and send it to the sending portion 20 as an amplified walking electrification change signal S1 via the amplifier 14.

In this case, since strength change of the walking quasi-electrostatic field HSE formed in response to a walking motion of a user appears at an infrasonic frequency band, the electric field detection portion 10 is able to accurately detect the strength change substantially without being influenced by noises such as hum noises.

The electric field detection portion 10 contacts the dielectric 13 directly with the user's epidermis OS and thereby can detect the strength change of the walking quasi-electrostatic field HSE with high sensitivity. Furthermore, by forming the dielectric 13 with soft vinyl chloride with a high permittivity, for example, the strength change can be detected with more sensitivity.

In addition to the above configuration, the electric field detection portion 10 is provided with a conductive case 15 surrounding the periphery of the FET 11 in condition that the conductive case 15 is electrically separated from the FET 11, and thereby detection other than the detection of the walking quasi-electrostatic field HSE of the user can be avoided to the utmost extent.

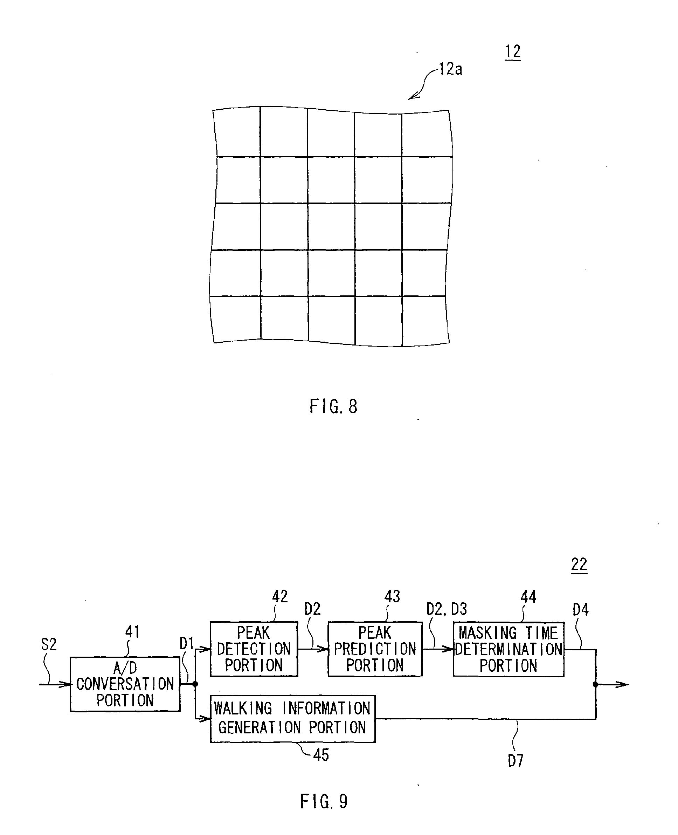

Furthermore, in the electric field detection portion 10, as shown in Figure 8 for example, the electrode surface 12a of the detection electrode 12 faced with the user' s epidermis OS is divided in multiple microelectrodes with almost the same shape and the same size, and an FET and an amplifier (not shown) are connected to each of the divided microelectrodes in the same connection condition as the FET 11 and the amplifier 14 of the detection electrode 12 and in a different route from the route to the FET 11.

Thus, in the electric field detection portion 10 (Figure 7), the strength change of the walking quasi-electrostatic field HSE formed on the user's epidermis OS, which is caused by a walking motion of the user, is sent to a low-pass filter (hereinafter referred to as an LPF) 22 via each microelectrode as an amplified walking electrification change signal S1, and the strength change is sent to a tissue information generation portion 21 via each microelectrode as local amplified walking electrification change signals A1 to An.

The walking motion described in this embodiment means a movement of walking on a flat passage surface without being especially conscious of the speed.

The sending portion 20 comprises the tissue information generation portion 21, the LPF 22, a waveform processing portion 23 and a modulation circuit 24. It inputs the amplified walking electrification change signal A1 to An from among the amplified walking electrification change signal S1 and the amplified walking electrification change signals Al to An supplied by the electric field detection portion 10 to the tissue information generation portion 21, and inputs the amplified walking electrification change signal S1 to the LPF 22.

As described above with reference to Figure 5, the amplified walking electrification change signals A1 to An are potentials of the human body detected by respective microelectrodes as relative potential differences, showing a potential difference pattern indicating the characteristics of the living tissue including the subepidermal tissue and veins below the detection electrode 12.

The tissue information generation portion 21 performs analog-digital conversion processing on the amplified walking electrification change signals A1 to A based thereon to digitalize them, generates the subepidermal tissue pattern under the detection electrode 12 as two-dimensionally represented tissue information D5 based on each of the digitalized amplified walking electrification change data, and sends it to the modulation circuit 24.

The LPF 22 abstracts a component with a low frequency at 20 Hz or below, for example, from the amplified walking electrification change signal S1 supplied by the amplifier 14 and sends it to the waveform processing portion 23 as a walking electrification change signal S2.

As shown in Figure 9, the waveform processing portion 23 comprises an A/D (analog/digital) conversion portion 41, a peak detection portion 42, a peak prediction portion 43, a masking time determination portion 44 and a walking information generation portion 45. The waveform processing portion 23 digitalizes the walking electrification change signal S2 supplied by the LPF 22 with the A/D conversion portion 41 and sends resultant walking electrification change data D1 to the peak detection portion 42 and the walking information generation portion 45.

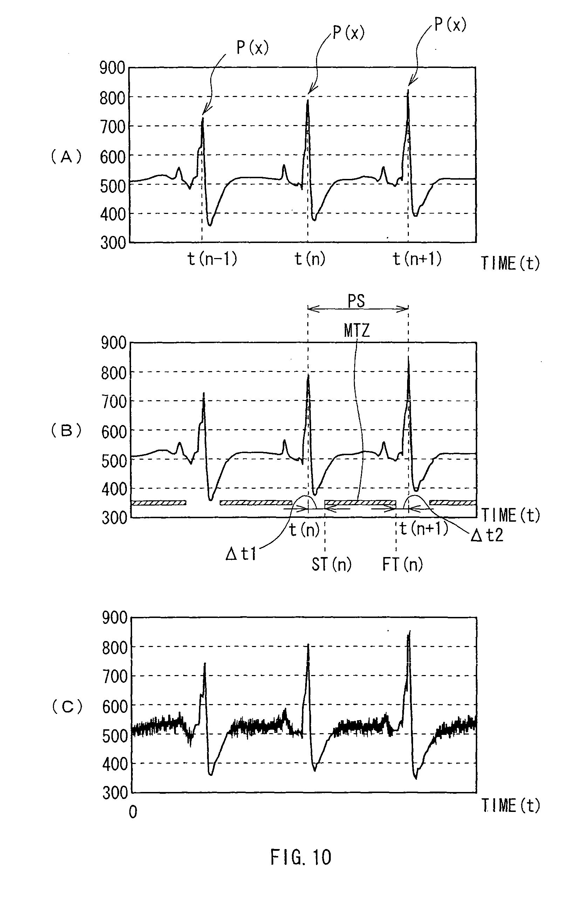

As shown in Figure 10 (A) , the peak detection portion 42 monitors the band of 8 Hz±2 Hz in the electrification change waveforms in the walking electrification change data D1 supplied by the A/D conversion portion 41 and detects an 8 Hz peak Px which appears in this band.

When detecting the 8 Hz peak Px, the peak detection portion 42 generates the time when the 8 Hz peak Px has been detected (hereinafter referred to as the current time) t(n) based on the clock in the card device 3 as current time data D2 and sends it to the peak prediction portion 43.

The peak prediction portion 43 holds the time (hereinafter the time is called as past time) t (n-1) of a past 8 Hz peak Px stored in an internal memory (not shown) , which had appeared immediately before the 8 Hz peak Px which appeared at the current time t (n) , and predicts, based on the current time t(n) and the past time t(n-1), the time (hereinafter referred to as future time) t (n+ 1) of a future 8 Hz peak Px which will appear immediately after the 8 Hz peak Px which appeared at the current time by adding the difference between the current time t(n) and the past time t(n-1) to the current time t(n) as represented by the following formula:

$$t (n + 1) = t (n) + ( t (n) - t (n - 1) ) \tag{15}$$

The peak prediction portion 43 generates the future time t (n+1) as predicted time data D3 and sends the predicted time data D3 and the current time data D2 to the masking time determination portion 44.

As shown in Figure 10 (B) , the masking time determination portion 44 determines the time zone (hereinafter referred to as a masking time zone) MTZ to be modulated by the modulation circuit 24 (Figure 7) on the subsequent stage by calculating a start time ST(n) and a finish time FT(n) of the masking time zone MTZ.

Specifically, the masking time determination portion 44 presets in advance a period (hereinafter referred to as a predicted peak decreasing period) Δt1 which begins when the 8 Hz peak Px appears and ends when a predetermined amplitude level is reached, and calculates the start time ST(n) of the masking time zone MTZ in accordance with the following formula:

$$S T (n) = t (n) +\Delta t 1 \tag{16}$$

The masking time determination portion 44 also presets in advance a period (hereinafter referred to as a predicted peak increasing period) Δt2 which begins at a predetermined amplitude level and ends when the 8 Hz peak Px appears, and calculates the finish time FT (n) of the masking time zone MTZ in accordance with the following formula:

$$F T (n) = t (n + 1)-\Delta t 2 \tag{17}$$

In this way, by removing the predicted peak decreasing period Δt1 and the predicted peak increasing period Δt2 preset in advance from the interval (hereinafter referred to as an 8 Hz peak interval) PS between an 8 Hz peak Px and the 8 Hz peak Px which appears immediately after the 8 Hz peak Px), the masking time determination portion 44 determines the masking time zone MTZ, in which the 8 Hz peak Px is avoided, and sends it to the modulation circuit 24 (Figure 7) as masking time data D4.

The walking information generation portion 45 acquires the walking electrification change data D1 supplied by the A/D conversion portion 41, for a predetermined time period, recognizes all the 8 Hz peaks Px (Figure 10(A)) which appear in the electrification change waveform in the walking electrification change data D1 corresponding to the predetermined time period, and removes such 8 Hz peak intervals PS that are beyond a predetermined allowable range in comparison with peak-interval average-width information stored in advance in the internal memory, from among the 8 Hz peak intervals PS of the recognized 8 Hz peaks Px (Figure 10(B)).

In this case, since the 8 Hz peak Px is set as an index, the walking information generation portion 45 is able to accurately leave the 8 Hz peak intervals PS corresponding to steady walking motion portions.

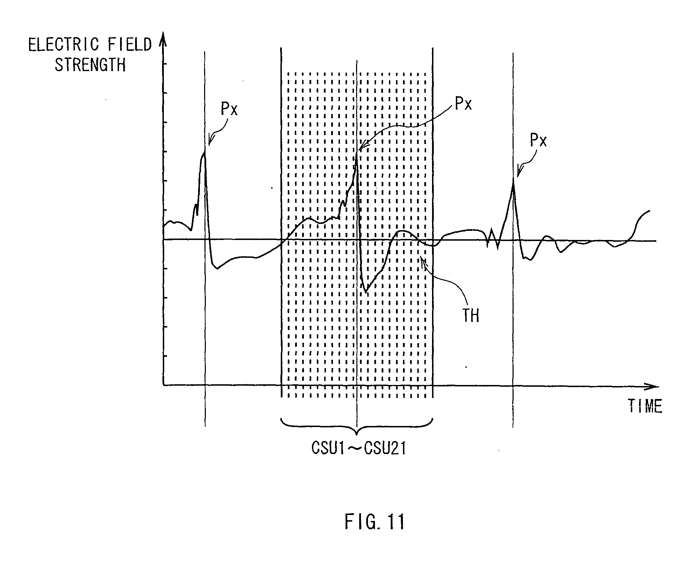

Then, the walking information generation portion 45 cut outs a portion corresponding to the portion from the center position of the 8 Hz peak Px to the immediate position between the 8 Hz peak Px and the 8 Hz peak Px immediately before the 8 Hz peak Px plus the portion from the center position of the 8 Hz peak Px to the immediate position between the 8 Hz peak Px and the 8 Hz peak Px immediately after the 8 Hz peak Px, as a one-step waveform TH, as shown in Figure 11.

In this case, since the 8 Hz peak Px is also set as an index, the walking information generation portion 45 is able to accurately cut out the portion as a one-step waveform TH corresponding to an actual one step in a walking motion.

The walking information generation portion 45 divides the one-step waveform TH into, for example, twenty-one subdivided sections CSU1 to CSU21 with almost equal intervals, in the time axis direction; integrates and normalizes each of the amplitude values (values indicating electrification change strength) for each of the divided subdivision sections CSU1 to CSU21; generates resultant twenty-one integral values as walking information D7 indicating the characteristics (walking pattern) of each portion in the one-step waveform TH; and sends it to the modulation circuit 24 (Figure 7).

The modulation circuit 24 performs data modulation processing on ID (IDentifier) information D6 stored in a memory (not shown) in the card device 3 in advance when the card device 3 was manufactured, for example, then performs data modulation processing on the tissue information D5 supplied by the tissue information generation portion 21, and finally performs data modulation processing on the walking information D7 supplied by the walking information generation portion 45. The modulation circuit 24 is able to change the order of data on which a data demodulation process should be performed, as required, based on a predetermined setting operation.

Specifically, the modulation circuit 24 performs data modulation processing on the ID information D6 (the tissue information D5 or the walking information D7) with a predetermined modulation method to generate a modulated signal HS with a high frequency, and applies the modulated signal HS to an electrification induction electrode 31 only during the masking time zone MTZ in the masking time data D4 supplied by the masking time determination portion 44.

The electrification induction electrode 31 oscillates according to the frequency of the modulated signal HS supplied by the modulation circuit 24 only during the masking time zone MTZ, and a quasi-electrostatic field (modulated signal HS) is generated from the electrification induction electrode 31 according to the oscillation.

Such quasi-electrostatic field (modulated signal HS) causes the user to be electrified only during the masking time zone MTZ according to the oscillation (modulated signal HS) of the electrification induction electrode 31 and thereby act as an antenna, and as shown in Figure 10(C), a quasi-electrostatic field according to the oscillation (hereinafter referred to as an information-transmission quasi-electrostatic field) DSE (Figure 6) isotropically spreads around the surface of the user.

As described above, in the sending portion 20, by changing the electrification condition of a user, the user is caused to act as an antenna, and as the result, an information-transmission quasi-electrostatic field DSE is formed on which the ID information D6 (the tissue information D5 or the walking information D7) is superimposed.

In this case, in the sending portion 20, the user is electrified during the masking time zone MTZ (Figure 10), in which the 8 Hz peak Px which appears with the highest strength in the strength change in the walking quasi-electrostatic field HSE is avoided, so that the ID information D6 superimposed on the information-transmission quasi-electrostatic field DSE can be prevented from being destroyed by the 8 Hz peak Px.

If the relative permittivity of the air is represented by 1, the wavelength in the air is represented by $\lambda$, the maximum distance for communication between the card device 3 and the authentication device 2 is represented by r, and the frequency of the modulated signal HS to be supplied to the electrification induction electrode 31 is represented by f, then the sending portion 20 is able to propagate, from the electrification induction electrode 31 to the user, a quasi-electrostatic field oscillating in accordance with the frequency f which satisfies the following formula:

$$f < \frac{c}{2 \pi \cdot r} \tag{18}$$

which is obtained by substituting the formula (10) into the formula (12) described above and rearranging the resultant formula.

Accordingly, when performing near field communication by causing a user who is passing through the entrance/exit passage portion 4 to act as an antenna, the sending portion 20 is able to form the communication space as space (substantially closed space) where a non-propagating information-transmission quasi-electrostatic field DSE (Figure 6) is always dominant, as described above with reference to Figures 3 and 4, and as a result, the communication output can be weakened to the extent that the communication contents are not propagated outside the communication space, and therefore, confidentiality of the communication contents can be secured more sufficiently.

The sending portion 20 is actually adapted to perform a sending process at the tissue information generation portion 21, the LPF 22, the waveform processing portion 23 and the modulation circuit 24 as software, in accordance with a predetermined sending program, under the control of a control portion not shown. The procedure for the sending process will be now described using the flowchart below.

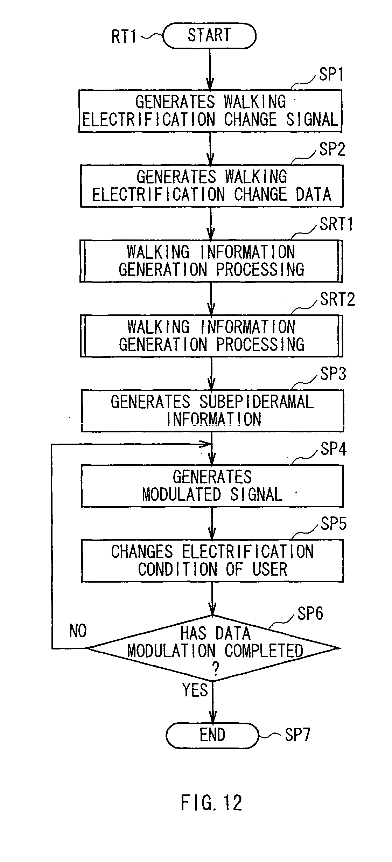

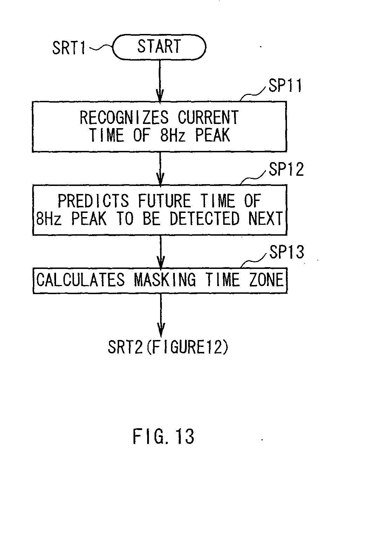

As shown in Figure 12, the sending portion 20 proceeds from the start step of a routine RT1 to the next step SP1, where it abstracts a low-frequency component of an amplified walking electrification change signal S1 supplied by the electric field detection portion 10 to generate a walking electrification change signal S2 and proceeds to the next step SP2.

At step SP2, the sending portion 20 performs analog-digital conversion based on the walking electrification change signal S2 to generate walking electrification change data D1, and proceeds to a masking time determination processing routine SRT1.

As shown in Figure 13, at step SP11, the sending portion 20 detects the 8 Hz peak Px (Figure 10 (A) ) based on the walking electrification change data D1 generated at step SP2 (Figure 12), and after recognizing the current time t(n) thereof, it proceeds to the next step SP12.

At step SP12, the sending portion 20 predicts the future time t (n+1 ) of an 8 Hz peak Px which will be detected next to the 8 Hz peak Px detected at step SP11 from the above formula (15), and proceeds to the next step SP13.

At step SP13, the sending portion 20 determines the masking time zone MTZ by calculating the start time ST (n) and the finish time FT (n) from the above formulas (16) and (17), based on the current time t(n) recognized at step SP11 and the future time t(n+1) predicted at step SP12, and proceeds to the next walking information generation processing routine SRT2 (Figure 12).

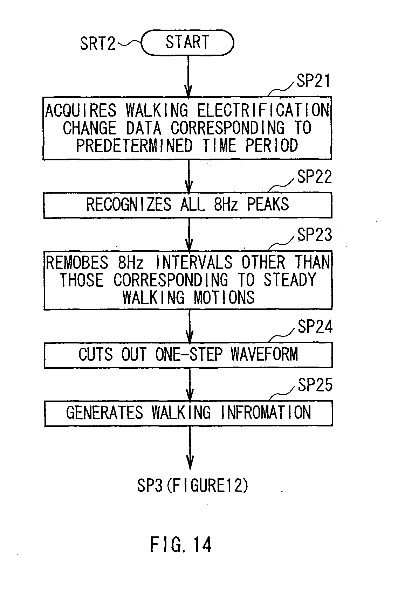

As shown in Figure 14, at step SP21, the sending portion 20 acquires the walking electrification change data D1 generated at step SP2 (Figure 12), corresponding to a predetermined time period, and then proceeds to step SP22.

At step SP22, the sending portion 20 recognizes all the 8 Hz peaks Px (Figure 10 (A) ) appearing in the walking electrification change data D1 corresponding to a predetermined time period, acquired at step SP21, and then proceeds to the next step SP23.

At step SP23, the sending portion 20 removes 8 Hz peak intervals PS that correspond to movements other than steady walking motions, such as walking motions performed when starting or finishing walking and movements performed when stopping walking, from among the 8 Hz peak intervals PS (Figure 10(B)) of the 8 Hz peaks Px recognized at step SP22, and then proceeds to the next step SP24.

At step SP24, the sending portion 20 cut outs a one-step waveform TH (Figure 11) from the waveform in the walking electrification change data D1 corresponding to a steady walking motion portion, and then proceeds to the next step SP25.

At step SP25, the sending portion 20 divides the one-step waveform TH cut out at step SP24 into, for example, twenty-one subdivided sections CSU1 to CSU21; integrates and normalizes the amplitude values of the subdivided sections CSU1 to CSU21 to generate walking information D7; and then proceeds to the next step SP3 (Figure 12).

At step SP3, the sending portion 20 generates tissue information D5 based on the amplified walking electrification change signals A1 to An supplied by the electric field detection portion 10, and then proceeds to the next step SP4.

At step SP4, the sending portion 20 performs data modulation processing on the ID information D6 supplied from the memory in the card device 3, the tissue information D5 generated at step SP3, or the walking information D7 generated by the walking information generation processing routine SRT2 to generate a modulated signal HS, and then proceeds to the next step SP5.

At step SP5, by applying the modulated signal HS generated at step SP4 to the electrification induction electrode 31 to change the electrification condition of the user during the masking time zone MTZ determined by the masking time determination processing routine SRT1, the sending portion 20 modulates the walking quasi-electrostatic field HSE (Figure 6) formed in the neighborhood of the user (Figure 10(C)), and then proceeds to the next step SP6.

In this case, the information-transmission quasi-electrostatic field DSE (Figure 6) formed isotropically around the surface of the user is acquired by the authentication device 2.

At step SP6, the sending portion 20 determines whether or not the data modulation processing has been completed at step SP4. If it has not been completed yet, the sending portion 20 returns to step SP4 and performs the data modulation processing again. On the contrary, if it has been completed, the sending portion 20 proceeds to the next step SP7 and ends the sending process.

As described above, in the sending portion 20, by changing the electrification condition of a user only during the masking time zone MTZ (Figure 10), in which the 8 Hz peak Px appears with the highest strength in the strength change in the walking quasi-electrostatic field HSE is avoided, it is possible to cause the user to act as an antenna and form an information-transmission quasi-electrostatic field DSE (Figure 6) in the neighborhood of the user while avoiding the modulated signal HS is destroyed by the 8 Hz peak Px.

(2-3) Configuration of an authentication device

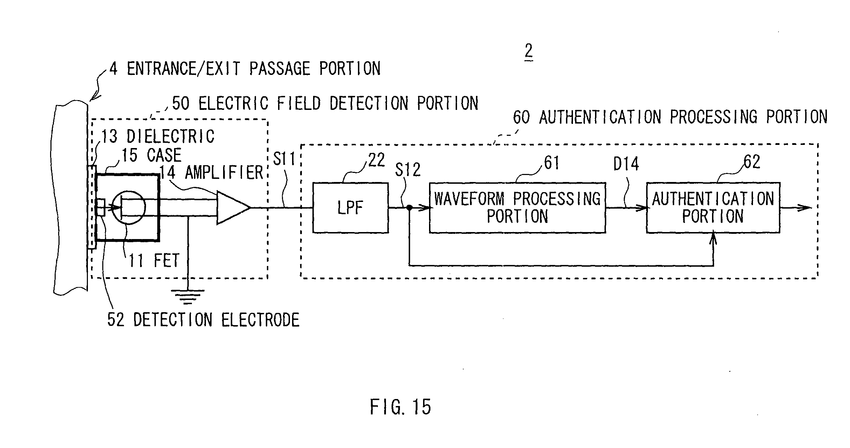

As shown in Figure 15, the authentication device 2 comprises an electric field detection portion 50 and an authentication processing portion 60.

The electric field detection portion 50 is provided on the internal surface of the entrance/exit passage portion 4 at the entrance side, for example, and comprises a detection electrode 52 without such microelectrodes as described above with reference to Figure 8 (that is, with the electrode surface not divided) instead of the detection electrode 12 in the electric field detection portion 10 (Figure 7) of the card device 3. Except for this, the configuration is the same as that of the electric field detection portion 10.

The authentication processing portion 60 comprises an LPF 22 similar to that in the sending portion 20 (Figure 7) of the card device 3, a waveform processing portion 61 with the same configuration as that of the waveform processing portion 23 without the walking information generation portion 45 (Figure 9), and an authentication portion 62.

The authentication device 2 detects strength change in an information-transmission quasi-electrostatic field DSE (a walking quasi-electrostatic field HSE) formed in the neighborhood of a user coming near to the entrance/exit passage portion 4 to pass through it, via the electric field detection portion 50 and the amplifier 14 sequentially as an amplified walking electrification change signal S11, almost at the same time as the card device 3 does; abstracts only a low-frequency component with the LPF 22; and sends it as an electrification change signal S12 to the waveform processing portion 61 and the authentication portion 62.

In this case, the waveform processing portion 61 performs each of the processings similar to those described above with reference to Figure 13 based on the electrification change signal S12 at the same time the processes are performed by the card device 3, and after that, it determines the masking time zone MTZ corresponding to the same time zone of the card device 3, and then sends it to the authentication portion 62 as masking time data D14.

The authentication portion 62 performs a predetermined authentication process based on the masking time data D14 supplied by the waveform processing portion 61 and the electrification change signal S12 supplied by the LPF 22, using an ID list prestored in an internal memory (not shown), registrant tissue information and registrant walking information registered in the internal memory in advance by a predetermined registration process.

The registration process is performed during a walking motion of the user with a detection electrode (not shown) of a predetermined registration device attached to the same portion of the user's arm where the detection electrode 12 (Figure 7) of a card device 3 supplied to the user is to be attached, in contact therewith, when a card device 3 is supplied to a user, for example.

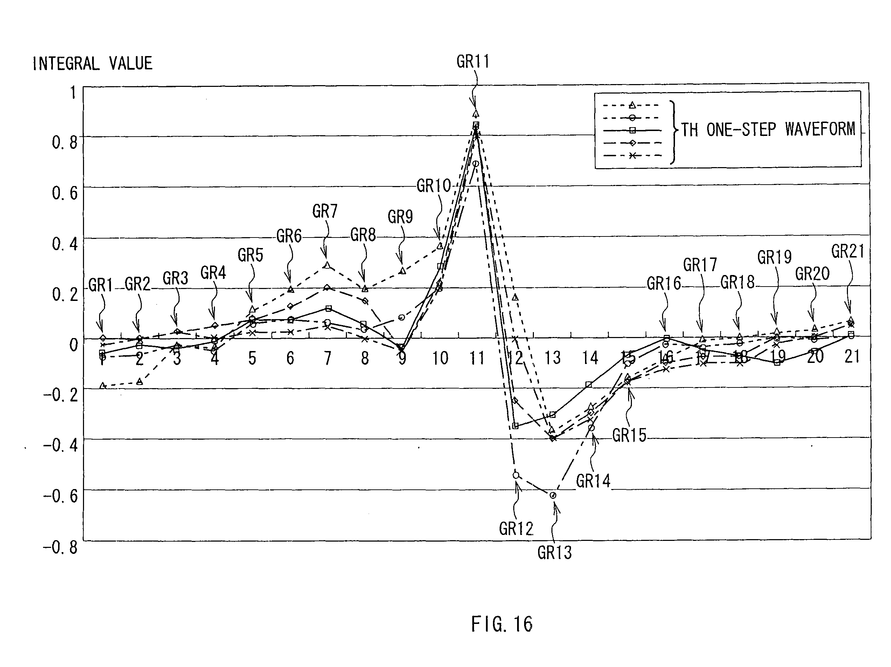

In this case, the registration device generates registrant tissue information that two-dimensionally shows the tissue pattern under the epidermis under the detection electrode and registers it in the internal memory of the authentication device 2, similarly to the card device 3. Furthermore, as shown in Figure 16, for example, the registration device divides each of one-step waveforms TH for five steps into twenty one subdivided sections CSU1 to CSU21 (Figure 11), and then registers a set of twenty one integral values for the subdivided sections CSU1 to CSU21 (hereinafter referred to as a group of registered parameters) obtained as a result of integration and normalization, in the internal memory of the authentication device 2 as registrant walking information indicating walking characteristics of the registrant, similarly to the card device 3.

As the authentication process, the authentication portion 62 first performs demodulation processing on the electrification change signal S12 supplied by LPF 22 in accordance with a predetermined demodulation method only during the masking time zone MTZ in the masking time data D14, and abstracts the ID information D6, the tissue information D5 or the walking information D7 superimposed on the electrification change signal S12 (the information-transmission quasi-electrostatic field DSE).

The authentication portion 62 then checks the ID list stored in the internal memory against the ID information D6 as the first stage. And then, only when there is information corresponding to the ID information D6 in the ID list, it checks the registrant tissue information stored in the internal memory against the tissue information D5 as the second stage. And then, only when there is registrant tissue information corresponding to the tissue information D5, it checks the registrant walking information stored in the internal memory against the walking information D7 as the third stage. And then, only when there is registrant walking information corresponding to the walking information D7, it opens the exit door 5 of the entrance/exit passage portion 4 (Figure 7).

An authentication processing portion 60 is actually adapted to perform the authentication process by the LPF 22, the waveform processing portion 61 and the authentication portion 62 as software, in accordance with a predetermined sending program, under the control of a control portion not shown. The procedure for the authentication process will be now described using the flowchart below.

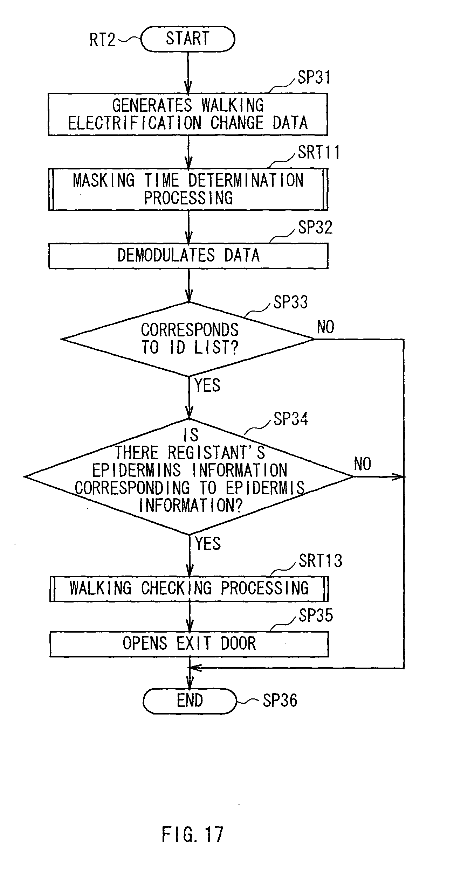

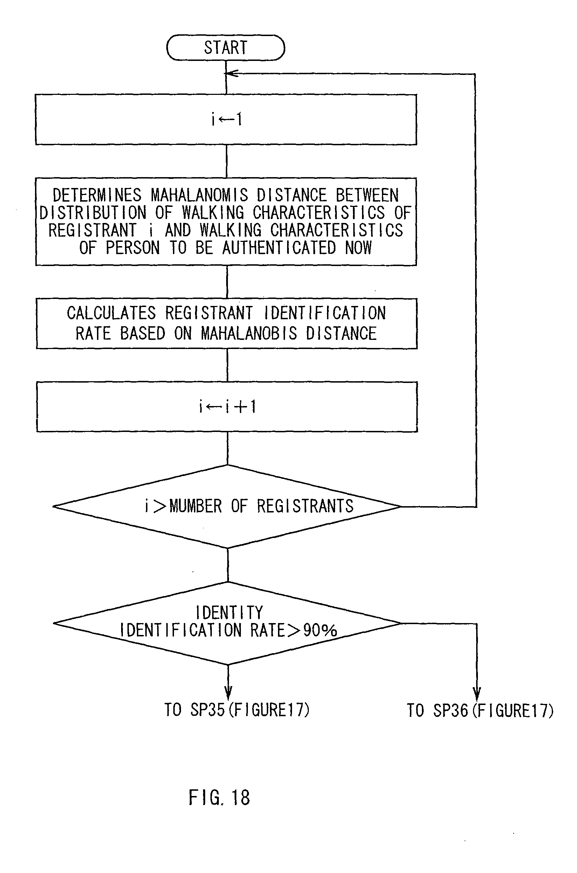

As shown in Figure 17, the authentication processing portion 60 proceeds from the start step of the routine RT2 to the next step SP31, and generates electrification change data by performing each of the same processings as performed at the steps SP1 and SP2 (Figure 12) by the card device 3 described above, on the amplified walking electrification change signal S11 detected and supplied by the electric field detection portion 50 at the same time when the card device 3 does, and then proceeds to the next masking time determination processing routine SRT11.

In the masking time determination processing routine SRT11, the authentication processing portion 60 determines a masking time zone MTZ by performing each of the same processings of the masking time processing routine SRT1 (Figure 13) for the card device 3, on the electrification change data generated at step SP31, and then proceeds to the next step SP32.

At step SP32, the authentication processing portion 60, by performing data demodulation processing on the electrification change data generated at step SP31 during the masking time zone MTZ determined by the masking time determination processing routine SRT11, abstracts the ID information D6, the tissue information D5 or the walking information D7 superimposed on the electrification change data, and then proceeds to the next step SP33.

At step SP33, the authentication processing portion 60 checks the ID information D6 abstracted at step SP32 against the ID list prestored in the internal memory, and determines whether or not there is information corresponding to the ID information D6 in the ID list.

If there is no such information, this indicates that the device is not the card device 3 supplied by the company but a fake device. In this case, the authentication processing portion 60 proceeds to the next step SP36 and ends the authentication process.

On the contrary, if such information exists, this indicates that the device is the card device 3 supplied by the company. In this case, the authentication processing portion 60 proceeds to the next step SP34.

At step SP34, the authentication processing portion 60 checks the tissue information D5 abstracted at step SP32 against the registrant tissue information registered in the internal memory in advance to determine wither or not there is registrant tissue information corresponding to the tissue information D5.

If there is no registrant tissue information, this indicates that the user having the card device 3 is not a person related to the company. In this case, the authentication processing portion 60 proceeds to the next step SP36 and ends the authentication process.

On the contrary, if such registrant tissue information exists, this indicates there is a high possibility that the user having the card device 3 is a person related to the company. In this case, the authentication processing portion 60 proceeds to the next walking checking processing routine SRT13.

At step SP41 in Figure 18, the authentication processing portion 60 determines, for the set of integral values obtained from a one-step waveform detected at step SP32, the Mahalanobis' distance from the center of distribution of registered parameters which are already registered with the authentication processing portion. The Mahalanobis' distance is determined for all the registrants.

Practically, the shorter the Mahalanobis' distance is, the higher registrant identification rate the authentication processing portion 60 calculates, and the longer the Mahalanobis' distance is, the lower registrant identification rate it calculates.

At step SP43, the authentication processing portion 60 determines whether or not any of the multiple registrant identification rates calculated at step SP42 is equal to or above 90%. If less than 90%, this indicates that the correspondence rate of the one step of the user relative to the registrant's step registered in the internal memory is low, that is, the user is not the registrant himself. In this case, the authentication processing portion 60 identifies that the user is not the registrant, and it proceeds to the next step SP36 and ends the authentication process.

On the contrary, if 90% or greater, this indicates that the user is the registrant himself. In this case, the authentication processing portion 60 recognizes that the user is a person related to the company and proceeds to the step SP35 (Figure 17).

At step SP35, the authentication processing portion 60 opens the exit door 5 (Figure 6) of the entrance/exit passage portion 4, and after that, it proceeds to the next step SP36 and ends the authentication process.

As described above, the authentication processing portion 60 not only determines the identity of the card device 3 based on the ID information D6 but also determines the identity of the user based on the tissue information D5 (living tissue pattern) and the walking information D7 (walking pattern), and thereby it can securely identify the relation between the card device 3 and the user without performing special processing such as encryption processing on the tissue information D5 to D7 on the side of the card device 3. Thus, the authentication processing portion 60 can even prevent a third person who has stolen a card device 3 from passing through the entrance/exit passage portion 4 instead of the user (a person related to a company).

(2-4) Auxiliary means in near field communication

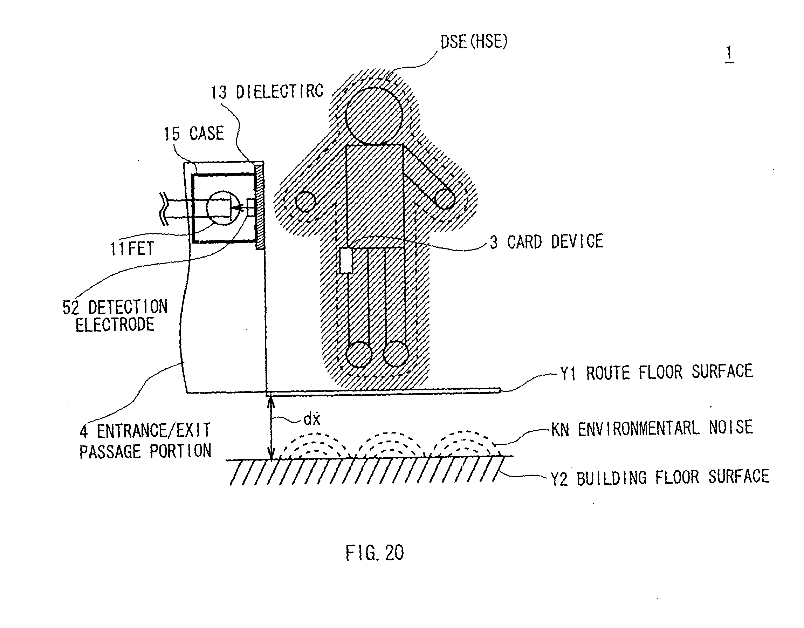

In addition to the above configuration, as shown in Figure 20, the authentication system 1 is provided with a floor surface (hereinafter referred to as a route floor surface) Y1 of the entrance/exit passage portion 4 in such a condition that it is not grounded to the ground (hereinafter referred to as a building floor surface) Y2 but is separated from the building floor surface Y2 by predetermined space dx (a gap).

In this case, the electrostatic capacity between the feet of the user and the building floor surface Y2 can be reduced to be less than the electrostatic capacity between the user and the side-surface electrode 7 by the amount corresponding to the space dx between the route floor surface Y1 and the building floor surface Y2, and thereby leakage of the information-transmission quasi-electrostatic field DSE (walking quasi-electrostatic field HSE) from the feet to the building floor surface Y2 can be prevented.

In addition to this, it is also possible to prevent noises (hereinafter referred to as environmental noises) KN caused by inconsistency of the building floor surface Y2, such as electrical discharge noises caused by electrically unstable condition due to a gap between joint surfaces of steel material in the building floor surface Y2 or rust of the steel material, from being induced from the route floor surface Y1 to the user.

Thus, in the communication system, it is possible to form, in a more stable condition, the equipotential surface of the information-transmission quasi-electrostatic field DSE (walking quasi-electrostatic field HSE) which is formed substantially isotropically from around the surface of the user when the user is electrified and the electrification change momentarily conducts over the periphery of the surface of the user, and therefore it is possible to stable near field communication.

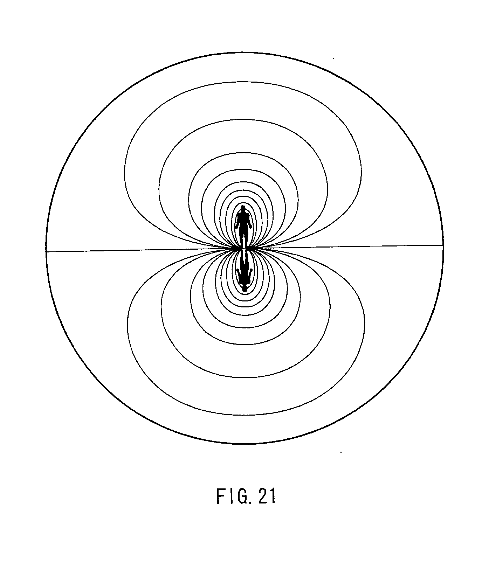

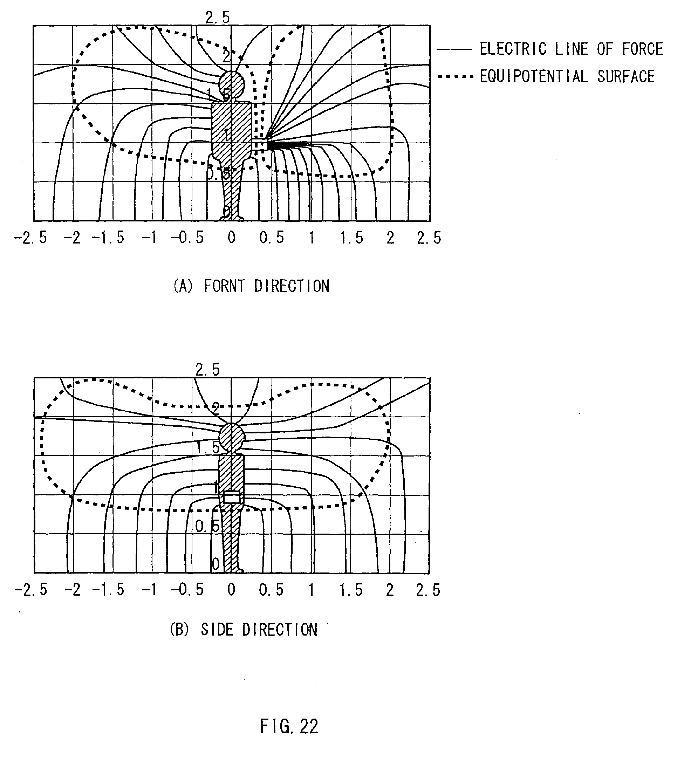

This will be visually apparent from comparison of Figure 21 showing the equipotential surface of a quasi-electrostatic field when a human body functions as an ideal dipole antenna and Figure 22 showing the results of experiments according to the present embodiment.

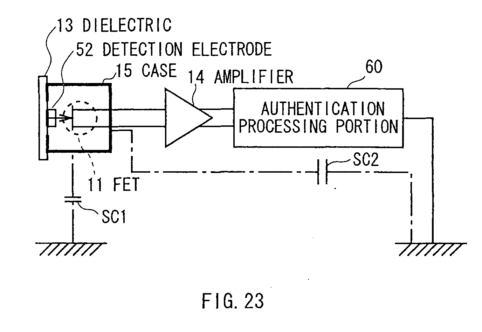

Furthermore, as shown in Figure 23, the authentication device 2 of the authentication system 1 is adapted to prevent leakage of a signal on the route from the detection electrode 52 to the authentication processing portion 60 via the FET 11 and the amplifier 14. Specifically, first, a conductive case 15 is electrically separated from the FET 11; and second, only the authentication processing portion 60 is connected to the ground on the receiving route.

Third, as means for preventing such leakage, the authentication device 2 is adapted to reduce the electrostatic capacity SC1 between the FET 11 and the ground in comparison with the electrostatic capacity SC2 on the route from the FET 11 to the ground via the authentication processing portion 60, for example, by increasing the interval (height) between the FET 11 and the ground.

Thus, the authentication device 2 can efficiently induce the information-transmission quasi-electrostatic field DSE (walking quasi-electrostatic field HSE) detected by the detection electrode 52 to the authentication processing portion 60 via the FET 11, and thereby receive the information-transmission quasi-electrostatic field DSE formed around the user with high sensitivity.

(2-5) Operation and effect

In the communication system 1 with the above configuration, the tissue information D5 and the walking information D7 as living organism identifying information are detected from the detection electrode 52 via the FET 11, based on the walking quasi-electrostatic field HSE formed in the neighborhood of the user, during a walking motion of the user which is essentially required for the user to pass through the entrance/exit passage portion 4 (Figure 6)

Accordingly, in the authentication system 1, it is possible to obtain information with high identifiability specific to the user without controlling the movement of the user and without performing special processing such as encryption.

Furthermore, since the tissue information D5 and the walking information D7 are obtained from the same the detection electrode 52, the authentication system 1 can be miniaturized. Thereby, uncomfortable feeling of the user can be reduced by reduction of the area to be in contact with the human body.

In this situation, in the card device 3 in the authentication system 1, a quasi-electrostatic field modulated according to the field information D5 and the walking information D7 (ID information D6) specific to the user is generated to electrify the user, and the information is sent via the information-transmission quasi-electrostatic field DSE with confidentiality which is isotropically formed in the neighborhood of the user.

In this case, in the authentication system 1, the nature of a quasi-electrostatic field and the nature of a user (human body) are utilized, and by the card device 3 and the authentication device 2 detecting the 8 Hz peak Px common to human bodies from a movement pattern specific to the user and performing the same processing at the same time, the masking time zone MTZ is determined.

As described above, in the authentication system 1, during a walking motion of the user which is essentially required for the user to pass an entrance/exit passage portion 4 (Figure 7), a quasi-electrostatic field generated by electrification or walking of a user is used not only synchronize the sending and receiving sides but also acquire specific tissue information D5 and walking information D7. Furthermore, the tissue information D5 and the walking information D7 are sent and received with the quasi-electrostatic field formed around the user used as an antenna. Thereby, the efficiency of communication can be considerably enhanced.

The authentication system 1 not only determines the identity of the card device 3 but also determines the identity of the tissue information D5 (living tissue pattern) and the walking information D7 (walking pattern) specific to a user, and thereby even the relation between the card device 3 and the user can be securely identified without providing special processing such as encryption processing on the tissue information D5 to D7 on side of the card device 3.

According to the configuration described above, a quasi-electrostatic field is used not only to synchronize the sending and receiving sides but also to acquire the tissue information D5 and the walking information D7. Furthermore, the tissue information D5 and the walking information D7 are sent and received using the quasi-electrostatic field generated around the user as an antenna, and thereby, sending and receiving can be realized via the quasi-electrostatic field, without any directional restrictions in the neighborhood of the user, with conf identially secured, and without forcing the user to perform a predetermined movement, and even the relation between the device and the human body can be identified based on information specific to the human body. Thus, the degree of freedom in communication using a quasi-electrostatic field can be enhanced.

(3) Other embodiments

In the embodiment described above, description has been made on the case where the tissue information generation portion 21 for generating tissue information D5 based on amplified walking electrification change signals A1 to An supplied via the detection electrode 12 of the electric field detection portion 10 or the walking information generation portion 45 for generating walking information D7 based on an amplified walking electrification change signal S1 supplied by the detection electrode 12 is applied as living organism information generation means for generating living organism information specific to a human body. The present invention, however, is not limited thereto, and other various living information generation means for generating living organism information on various other living organisms such as fingerprints and cells can be applied.

In the embodiment described above, description has been made on the case where the 8 Hz peak Px is detected by the walking information generation portion 45 as walking information generation means, from the strength displacement of the walking quasi-electrostatic field HSE formed around a human body in response to a walking motion of the human body. The present invention, however, is not limited thereto, and the peak of the electric field displacement may be detected which is generated around the human body by various other bipedal motions such as brisk walking, up-and-down movement on stairs and stepping movement on the same place, that is, such movements that include a state in which the entire plantar surface of one foot is in contact with the ground and the tiptoe of the other foot has just left the ground.

In this case, the amplitude peak in the walking waveform changes according to the speed of the movement performed from when the right foot (left foot) completely gets in contact with the ground until when the tiptoe of the right foot (left foot) has just left the ground. Therefore, by detecting the amplitude peak that appears at the frequency band according to the movement speed from when the right foot (left foot) in a bipedal motion to be detected is completely in contact with the ground until when the tiptoe of the right foot (left foot) has just left the ground as an index instead of the 8 Hz peak, the effect similar to that of the embodiment described above can be obtained.

In this case, if the masking time determination portion 44 changes the predicted peak decreasing period $\Delta t1$ and the predicted peak increasing period $\Delta t2$ based on the frequency band according to the movement speed from when the right foot (left foot) in a bipedal motion to be detected is completely in contact with the ground until when the tiptoe of the right foot (left foot) has just left the ground, then near field communication can be performed in a more stable condition.

In the embodiment described above, description has been made on the case where the card device 3 as a sending device is positioned on a predetermined portion of a user' s arm in contact therewith. The present invention, however, is not limited thereto, and the card device 3 may be positioned on various other portions of the epidermis of the user in contact therewith. For example, it may be embedded in a stud earring.

Furthermore, in the embodiment described above, description has been made on the case where the card device 3 is formed in a card shape. The present invention, however, is not limited thereto, and the card device 3 may be formed in various other shapes. After all any shape may be possible only if it is of a mobile type.

Furthermore, in the embodiment described above, description has been made on the case where the route floor surface Y1 is provided for the entrance/exit passage portion 4 in a condition that it is separated from the building floor surface Y2 (Figure 20) by predetermined space dx. The present invention, however, is not limited thereto, and a member with a low relative permittivity may be filled in the space dx.

In this case, if the relative permittivity of the member filled between the route floor surface Y1 and the building floor surface Y2 is represented by $\varepsilon$, the gap between the route floor surface Y1 and the building floor surface the building floor surface Y2 is represented by dx, the permittivity of vacuum electric constant is represented by $\varepsilon0$, and the area of the user's soles is represented by S, then the electrostatic capacity CY2 between the user's feet and the building floor surface Y2 approximates the relation represented by the following formula:

$$C\,Y\,2 = \varepsilon_0 \cdot \varepsilon\, \frac{S}{d\,x} \tag{19}$$

Therefore, if the distance dx between the gate floor surface Y1 and the ground surface Y2 and the relative permittivity $\varepsilon$ of the member filled between the gate floor surface Y1 and the ground surface Y2 are selected in consideration of the above relation, the electrostatic capacity CY2 between the user's feet and the ground surface Y2 can be certainly reduced to be less than the electrostatic capacity between the user and the detection electrode 52. Thus, leakage of the information-transmission quasi-electrostatic field DSE (walking quasi-electrostatic field HSE) from the user's feet to the ground surface Y2 can be prevented more securely, and thereby near field communication can be stabilized more securely.

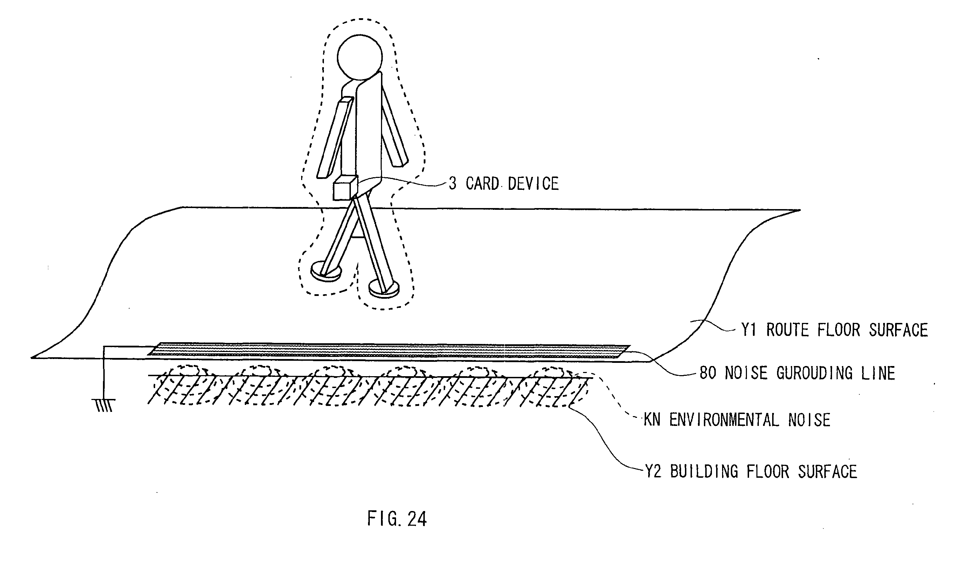

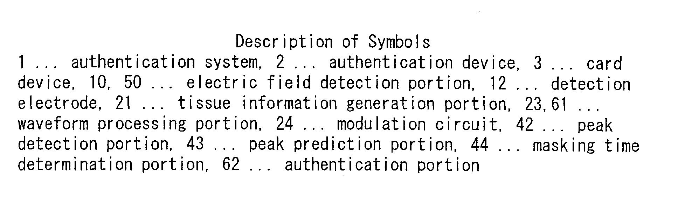

Furthermore, in the embodiment described above, description has been made on the case where the route floor surface Y1 is provided for the entrance/exit passage portion 4 in a condition that it is separated from the building floor surface Y2 (Figure 20) by predetermined space dx, as coupling preventing means for preventing a user and the ground from being electrically coupled with each other. The present invention, however, is not limited thereto, and there may be provided a noise absorption/grounding line 80 laid on the route floor surface Y1 and grounded to the building floor surface Y2, as shown in Figure 24.

In this case, it is possible to prevent such noises (hereinafter referred to as environmental noises) KN as are caused by inconsistency of the building floor surface Y2 from being induced from the route floor surface Y1 to the user and thereby stabilize the near field communication, similarly to the embodiment described above. Furthermore, if not only the space dx but also the noise absorption/grounding line 80 is provided between the route floor surface Y1 and the building floor surface Y2, stabilization of the near field communication can be enhanced more.

Furthermore, in the embodiment described above, description has been made on the case where electrification change of a user (an information-transmission quasi-electrostatic field DSE or a walking quasi-electrostatic field HSE) is detected by an FET 11 (FET connected to microelectrodes) as the amplified walking electrification change signal S1 (A1 to An). The present invention, however, is not limited thereto, and the change in the electrification condition of the user may be detected by various other detection means such as an induction-electrode-type field strength meter for measuring the voltage induced by induction voltage, an induction-electrode-type modulation-amplification-system field strength meter for AC converting a direct signal obtained by an induction electrode using a chopper circuit, oscillation capacity and the like, an electro-optic-effect-type field strength meter for applying en electric field to material having an electro-optic effect to measure change in the light propagation characteristics caused in the material, and, only for the card device 3, an electrometer, a shunt-resistor-type field strength meter, a current-collection-type field strength meter and the like.

Furthermore, in the embodiment described above, description has been made on the case where electrification induction means is realized by the modulation circuit 24 and the electrification induction portion 30. The present invention, however, is not limited thereto, and the electrification induction means may be realized by various other configurations.

Furthermore, in the embodiment described above, description has been made on the case where demodulation means is realized by the LPF 22 and the authentication portion 62. The present invention, however, is not limited thereto, and the demodulation means may be realized by various other configurations.

Furthermore, in the embodiment described above, description has been made on the case where a user is identified by the authentication portion 62 as identification means based on tissue information D5 and walking information D7 as living organism information. The present invention, however, is not limited thereto, and a user may be identified on one of the tissue information D5 and the walking information D7. Alternatively, a user may be identified by living organism information such as fingerprints combined with and added to the tissue information D5 and the walking information D7.

Furthermore, in the embodiment described above, description has been made on the case where near field communication is performed via one user between the card device 3 as a mobile-type sending device provided in the neighborhood of the user and the authentication device 2 as a receiving device provided on a predetermined control target. The present invention, however, is not limited thereto, and near field communication may be performed via multiple users. In this case, the same effect as that of the embodiment described above can be obtained.

Furthermore, in the embodiment described above, description has been made on the case where the present invention is applied to an authentication system 1 which opens an exit door 5 as necessary when a user enters or exits from an entrance/exit passage portion 4 as a communication route. The present invention, however, is not limited thereto and can be broadly applied to authentication systems for various other purposes, such as a communication system with a communication route in the neighborhood of the desk, for opening the door of a desk as necessary when a user comes near to the desk, a communication system with a communication route in the neighborhood of a personal computer, for powering on the personal computer when a user comes near to the personal computer, and a communication system using a conveyance passage for conveying a predetermined identification target as a communication route, for switching conveyance passages as necessary when the identification target is conveyed to a predetermined position, that is, to any authentication system that electrifies a human body according to living organism information specific to the human body to cause the human body to act as an antenna, on the sending side, and acquires identification information from a quasi-electrostatic field formed in the neighborhood of the human body to authenticate the human body, on the receiving side.

In this case, in the present invention, the authentication device 2 as a receiving device can be broadly applied to various other devices provided on or in the neighborhood of a video tape recorder, a television set, electronics such as a mobile telephone or a personal computer, medical equipment, an automobile, a desk, and other control targets to be controlled, for example. In this case, the same effect as that of the embodiment described above can be obtained.

Furthermore, in the embodiment described above, description has been made on the case where each of the processings by a sending portion 20 or an authentication processing portion 60 is realized by a program. The present invention, however, is not limited thereto, and a part or all of each processing may be realized by hardware means, such as an integrated circuit dedicated the processing.

Furthermore, in the embodiment described above, description has been made on the case where the sending process (Figure 12) or the authentication process (Figure 17) described above are performed in accordance with a program prestored in an internal memory. The present invention, however, is not limited thereto, and the sending process or the authentication process may be performed by mounting a program storage medium in which the program is stored into an information processor.

In this case, the program storage medium to have the program for executing the sending process or the authentication process installed and make it executable may be realized not only by a package media such as a flexible disk, a CD-ROM (Compact Disk-Read Only Memory), and a DVD (Digital Versatile Disc) but also by a semiconductor memory or a magnetic disk in which the program is temporarily or permanently stored. As means for storing an analysis program in such a program storage medium, a wired or wires communication medium, such as a local area network, the Internet, and digital satellite broadcasting, may be utilized, and the analysis program may be stored via various communication interfaces such as a router and a modem.

As described above, according to the present invention, in an authentication system comprising sending and receiving devices for sending and receiving information via a quasi-electrostatic field, the sending device detects living organism information specific to a human body and generates a quasi-electrostatic field modulated according to the living organism information to electrify the human body. Meanwhile, the receiving device demodulates the living organism information based on change in the electrification condition of the human body and identifies the human body based on the living organism information. Thereby, sending and receiving of information can be realized without any directional restriction in the neighborhood of a user, with confidentiality secured, and without forcing the user to perform a predetermined movement. Furthermore, even the relation between the device and the human body can be identified based on the information specific to the human body. Thus, the degree of freedom in communication with a quasi-electrostatic field can be enhanced.

Industrial Applicability

The present invention is adapted to an authentication system for performing authentication using living organism information specific to a human body, for example, in the case of opening a door provided for a predetermined entrance/exit passage when the human body enters or exits from the entrance/exit passage, in the case of unlocking a drawer provided for a desk as necessary when the human body comes near to the desk, and the like.

**Claims**

1. An authentication system comprising a sending device and a receiving device for sending and receiving information via a quasi-electrostatic field, **characterized in that**:

   the sending device comprises:

   living organism information generation means for generating living organism information specific to a human body; and
   electrification induction means for electrifying the human body by generating a quasi-electrostatic field modulated according to the living organism information; and
   the receiving device comprises:

   demodulation means for demodulating the living organism information based on change in the electrification condition of the human body; and
   identification means for identifying the human body based on the living organism information.

2. The authentication system according to claim 1, **characterized in that**:

   the living organism information generation means comprises:

   a detection electrode contacted with the epidermis of the human body, for detecting potential change on the surface of the human body faced with the contacted surface; and

tissue information generation means for generating tissue information indicating a tissue pattern under the portion of the epidermis of the human body faced with the contacted surface, as the living organism information based on the potential change detected by the detection electrode.

**3.** The authentication system according to claim 2, **characterized in that**:

the detection electrode comprises multiple microelectrodes electrically independent from one another; and

the tissue information generation means generates the tissue information based on relative potential difference in the potential change detected by each of the microelectrodes.

**4.** The authentication system according to claim 1, **characterized in that**:

the living organism information generation means comprises:

a detection electrode contacted with the epidermis of the human body, for detecting potential change on the surface of the human body faced with the contacted surface in response to a bipedal walking motion of the human body; and

walking information generation means for generating walking information indicating a walking pattern of the human body, based on the potential change detected by the detection electrode.

**5.** The authentication system according to claim 4, **characterized in that**:

the walking information generation means generates the walking information with the amplitude peak that appears at a particular frequency band in the potential change as an index.

**6.** The authentication system according to claim 1, **characterized in that**:

the living organism information generation means comprises:

a detection electrode contacted with the epidermis of the human body, for detecting potential change on the surface of the human body faced with the contacted surface in response to a bipedal walking motion of the human body;

walking information generation means for generating walking information indicating a walking pattern the human body, based on the potential change detected by the detection electrode; and

tissue information generation means for generating tissue information indicating a tissue pattern under the portion of the epidermis of the human body faced with the contacted surface, as the living organism information based on the potential change detected by the detection electrode.

**7.** An authentication method for sending and receiving information via quasi-electrostatic field, **characterized in** comprising:

on the sending side,

a living organism information generation step of generating living organism information specific to a human body; and

an electrification induction step of electrifying the human body by generating a quasi-electrostatic field modulated according to the living organism information; and

on the receiving side,

a demodulation step of demodulating the living organism information based on change in the electrification condition of the human body; and

an identification step of identifying the human body based on the living organism information.

**8.** A sending device for sending information via a quasi-electrostatic field, **characterized in** comprising:

living organism information generation means for generating living organism information specific to a human body; and

electrification induction means for electrifying the human body by generating a quasi-electrostatic field modulated according to the living organism information.

**9.** The sending device according to claim 8, **characterized in that**:

the living organism information generation means comprises:

a detection electrode contacted with the epidermis of the human body, for detecting potential change on the surface of the human body faced with the contacted surface; and
tissue information generation means for generating tissue information indicating a tissue pattern under the portion of the epidermis of the human body faced with the contacted surface, as the living organism information based on the potential change detected by the detection electrode.

**10.** The sending device according to claim 8, **characterized in that**:

the living organism information generation means comprises:

a detection electrode contacted with the epidermis of the human body, for detecting potential change on the surface of the human body faced with the contacted surface in response to a bipedal walking motion of the human body; and
walking information generation means for generating walking information indicating a walking pattern of the human body, based on the potential change detected by the detection electrode.

**11.** A receiving device for receiving information via a quasi-electrostatic field, **characterized in** comprising:

demodulation means for, based on change in the electrification condition of a human body electrified by a quasi-electrostatic field modulated according to living information specific to the human body, demodulating the living organism information; and
identification means for identifying the human body based on the living organism information.

FIG. 1

FIG. 2

FIG. 3

FIG. 4

FIG. 5

SL EPIDERMAL LAYER

BL DERMAL LAYER

HSE, DSE

1

2 AUTHENTICATION DEVICE

3 CARD DEVICE

4 ENTRANCE/EXIT PASSAGE PORTION

5 EXIT DOOR

FIG. 6

10 ELECTRIC FIELD DETECTION PORTION

3

~D6

20 SENDING PORTION

~A1～An

21

TISSUE INFORMATION
GENERATION PORTION

~D5

14 AMPLIFIER  S1

22  S2

23

24

LPF

WAVEFORM
PROCESSING PORTION

MODULATION
CIRCUIT

D4, D7

~HS

15 CASE

30 ELECTRIFICATION INDUCTION PORTION

11 FET

31 ELECTRIFICATION
INDUCIOTN ELECTORDE

32 DIELECTRIC

12 DETECTION ELECTODE

13 DIELECTRIC

OS USER'S EPIDERMIS

FIG. 7

12

12a

FIG. 8

22

S2 → [41 A/D CONVERSATION PORTION] → D1

D1 → [42 PEAK DETECTION PORTION] → D2 → [43 PEAK PREDICTION PORTION] → D2, D3 → [44 MASKING TIME DETERMINATION PORTION] → D4

D1 → [45 WALKING INFORMATION GENERATION PORTION] → D7

FIG. 9

FIG. 10

FIG. 11

RT1 — ( START )

↓

SP1

GENERATES WALKING
ELECTRIFICATION CHANGE SIGNAL

↓

SP2

GENERATES WALKING
ELECTRIFICATION CHANGE DATA

↓

SRT1

WALKING INFORMATION
GENERATION PROCESSING

↓

SRT2

WALKING INFORMATION
GENERATION PROCESSING

↓

SP3

GENERATES SUBEPIDERAMAL
INFORMATION

↓

SP4

GENERATES
MODULATED SIGNAL

↓

SP5

CHANGES ELECTRIFICATION
CONDITION OF USER

↓

NO

SP6

HAS DATA
MODULATION COMPLETED
?

YES

↓

( END ) — SP7

FIG. 12

31

```
 SRT1 ──( START )          ·

                           ┌─ SP11
        ┌─────────────────────────┐
        │  RECOGNIZES CURRENT     │
        │   TIME OF 8Hz PEAK      │
        └─────────────────────────┘

                           ┌─ SP12
        ┌─────────────────────────┐
        │  PREDICTS FUTURE TIME OF │
        │ 8Hz PEAK TO BE DETECTED NEXT│
        └─────────────────────────┘

                           ┌─ SP13
        ┌─────────────────────────┐
        │ CALCULATES MASKING TIME ZONE │
        └─────────────────────────┘

           SRT2 (FIGURE12)
```

FIG. 13

SRT2 — ( START )

|
↓

SP21
ACQUIRES WALKING ELECTRIFICATION
CHANGE DATA CORRESPONDING TO
PREDETERMINED TIME PERIOD

↓

SP22
RECOGNIZES ALL 8Hz PEAKS

↓

SP23
REMOBES 8Hz INTERVALS OTHER THAN
THOSE CORRESPONDING TO STEADY
WALKING MOTIONS

↓

SP24
CUTS OUT ONE-STEP WAVEFORM

↓

SP25
GENERATES WALKING INFROMATION

↓

SP3 (FIGURE12)

FIG. 14

4 ENTRANCE/EXIT PASSAGE PORTION

2

,50 ELECTRIC FIELD DETECTION PORTION

60 AUTHENTICATION PROCESSING PORTION

13 DIELECTRIC
15 CASE
14 AMPLIFIER
11 FET
52 DETECTION ELECTRODE

S11
22 S12
LPF
61
WAVEFORM PROCESSING PORTION
D14
62
AUTHENTICATION PORTION

FIG. 15

EP 1 598 009 A1

INTEGRAL VALUE

GR11

GR10

GR7
GR6
GR8 GR9

GR5
GR1 GR2 GR3 GR4

GR16 GR17 GR18 GR19 GR20 GR21

}TH ONE-STEP WAVEFORM

GR15

GR12 GR14

GR13

FIG. 16

EP 1 598 009 A1

RT2 — ( START )

SP31
GENERATES WALKING
ELECTRIFICATION CHANGE DATA

SRT11
MASKING TIME DETERMINATION
PROCESSING

SP32
DEMODULATES DATA

SP33
CORRESPONDS
TO ID LIST? — NO

YES

SP34
IS
THERE REGISTANT'S
EPIDERMINS INFORMATION
CORRESPONDING TO EPIDERMIS
INFORMATION? — NO

YES

SRT13
WALKING CHECKING PROCESSING

SP35
OPENS EXIT DOOR

( END ) — SP36

FIG. 17

FIG. 18

FIG. 19

FIG. 20

FIG. 21

(A) FORNT DIRECTION

(B) SIDE DIRECTION

FIG. 22

13 DIELECTRIC

52 DETECTION ELECTRODE

15 CASE    14 AMPLIFIER     60

AUTHENTICATION
PROCESSING PORTION

SC2

11 FET

SC1

FIG. 23

FIG. 24

- 3 CARD DEVICE
- Y1 ROUTE FLOOR SURFACE
- 80 NOISE GUROUDING LINE
- KN ENVIRONMENTAL NOISE
- Y2 BUILDING FLOOR SURFACE

Description of Symbols
1 ... authentication system, 2 ... authentication device, 3 ... card device, 10, 50 ... electric field detection portion, 12 ... detection electrode, 21 ... tissue information generation portion, 23,61 ... waveform processing portion, 24 ... modulation circuit, 42 ... peak detection portion, 43 ... peak prediction portion, 44 ... masking time determination portion, 62 ... authentication portion

**INTERNATIONAL SEARCH REPORT**

| International application No. |
| --- |
| PCT/JP2004/002378 |

A. CLASSIFICATION OF SUBJECT MATTER
Int.Cl⁷ A61B5/117

According to International Patent Classification (IPC) or to both national classification and IPC

B. FIELDS SEARCHED

Minimum documentation searched (classification system followed by classification symbols)
Int.Cl⁷ A61B5/117, H04B13/00

Documentation searched other than minimum documentation to the extent that such documents are included in the fields searched

| | | | |
| --- | --- | --- | --- |
| Jitsuyo Shinan Koho | 1922–1996 | Toroku Jitsuyo Shinan Koho | 1994–2004 |
| Kokai Jitsuyo Shinan Koho | 1971–2004 | Jitsuyo Shinan Toroku Koho | 1996–2004 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 11-509380 A (Massachusetts Institute of Technology), 17 August, 1999 (17.08.99), Full text; all drawings & EP 824799 B1 & US 5914701 A & WO 96/36134 A1 & DE 69623115 E & AU 9656713 A & CA 2220294 C & ES 2180767 T3 & KR 99008128 A & BR 9608465 A & MX 205475 B | 1–11 |
| P,A | JP 2003-331271 A (Sony Corp.), 21 November, 2003 (21.11.03), Full text; all drawings & WO 2003/96272 A1 | 1–11 |

| ☒ Further documents are listed in the continuation of Box C. | ☐ See patent family annex. |
| --- | --- |

\*      Special categories of cited documents:
"A"   document defining the general state of the art which is not considered to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
| --- | --- |
| 24 May, 2004 (24.05.04) | 08 June, 2004 (08.06.04) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**EP 1 598 009 A1**

| **INTERNATIONAL SEARCH REPORT** | International application No. |
|---|---|
| | PCT/JP2004/002378 |

C (Continuation).  DOCUMENTS CONSIDERED TO BE RELEVANT

| Category* | Citation of document, with indication, where appropriate, of the relevant passages | Relevant to claim No. |
|---|---|---|
| A | JP 3065356 B2 (Siemens AG.), 12 May, 2000 (12.05.00), Full text; all drawings & EP 833587 B1 & US 6228029 B1 & WO 97/37592 A1 & CN 1195278 A & BR 9706596 A & KR 99022764 A & MX 209314 B | 1-11 |
| A | JP 10-228524 A (International Business Machines Corp.), 25 August, 1998 (25.08.98), Full text; all drawings & EP 843425 B1 & US 5796827 A & DE 69719919 E & CN 1185065 A & ES 2189926 T & KR 272700 B & TW 353255 A | 1-11 |
| A | JP 3074644 B2 (Daimler Chrysler AG.), 09 June, 2000 (09.06.00), Full text; all drawings & US 5811897 A & GB 2308481 B & DE 19547560 C2 & FR 2742903 A1 & IT 1290155 B | 1-11 |
| A | JP 10-229357 A (Nippon Telegraph And Telephone Corp.), 25 August, 1998 (25.08.98), Full text; all drawings & US 6223018 B1 | 1-11 |
| A | Keisuke HACHISUGA et al., "Jintai o Densoro to shita Joho Tsushin Device no Kaihatsu", Micromechatronics, Journal of the Horological institute of Japan, 10 June, 2002 (10.06.02), Vol.46, No.2, pages 53 to 64 | 1-11 |
| A | Katsuyuki FUJII et al., "Jintai o Densoro to shita Tsushin System no Model ni Kansuru Kento", Eijogaku Giho, The Institute of Image Information and Television Engineers, , 03 March, 2002 (03.03. 02), Vol.26, No.20, pages 13 to 18 | 1-11 |

Form PCT/ISA/210 (continuation of second sheet) (January 2004)

46